# EUROPEAN PATENT SPECIFICATION

(11) **EP 1 786 908 B1**
(45) Date of publication and mention of the grant of the patent: **03.03.2010**
(21) Application number: 05772662.2
(22) Date of filing: 18.08.2005
(51) Int. Cl.: C12N 15/82, C12N 15/54, C12N 9/12, A01H 5/10, A21D 2/18, A23L 1/10, A23L 1/0522, C08B 30/00

(54) **PLANTS WITH INCREASED PLASTIDIC ACTIVITY OF R3 STARCH-PHOSPHORYLATING ENZYME**
PFLANZEN MIT ERHÖHTER PLASTIDÄR AKTIVITÄT DER STÄRKEPHOSPHORYLIERENDEN R3-ENZYME
VEGETAUX A ACTIVITE PLASTIDIQUE ACCRUE DE L'ENZYME R3 DE PHOSPHORYLATION DE L'AMIDON

(30) Priority: 18.08.2004 EP 04090316; 18.08.2004 US 602454 P
(43) Date of publication of application: 23.05.2007
(73) Proprietor: Bayer CropScience AG, 40789 Monheim (DE)
(72) Inventor: FROHBERG, Claus, 14532 Kleinmachnow (DE)
(74) Representative: Beyer, Andreas
(86) International application number: PCT/EP2005/009039
(87) International publication number: WO 2006/018319

(56) References cited:
- EP-B1- 0 656 951
- EP-B1- 0 688 362
- EP-B1- 1 021 535
- EP-B1- 1 131 444
- EP-B1- 1 157 100
- EP-B1- 1 240 313
- EP-B1- 1 246 841
- EP-B1- 1 290 160
- EP-B1- 1 320 542
- EP-B1- 1 594 963
- EP-B1- 1 600 510
- WO-A-00/77229
- WO-A-02/34923
- DATABASE Geneseq [Online] 17 October 2000 (2000-10-17), "Arabidopsis thaliana DNA fragment SEQ ID NO: 38779." XP002355632 retrieved from EBI accession no. GSN:AAC43304 Database accession no. AAC43304 -& DATABASE Geneseq [Online] 17 October 2000 (2000-10-17), "Arabidopsis thaliana protein fragment SEQ ID NO: 38782." XP002355633 retrieved from EBI accession no. GSN:AAG32188 Database accession no. AAG32188 -& EP 1 033 405 A (CERES INCORPORATED) 6 September 2000 (2000-09-06)
- DATABASE EMBL [Online] 17 February 2003 (2003-02-17), "Arabidopsis thaliana clone RAFL15-16-O10 (R20644) unknown protein (At4g24450) mRNA, complete cds." XP002355634 retrieved from EBI accession no. EM_PRO:BT004118 Database accession no. BT004118
- BLENNOW A ET AL: "Starch phosphorylation: A new front line in starch research" TRENDS IN PLANT SCIENCE, ELSEVIER SCIENCE, OXFORD, GB, vol. 7, no. 10, October 2002 (2002-10), pages 445-450, XP002339248 ISSN: 1360-1385

### SEQUENCE LISTING

<110> Bayer CropScience GmbH
<120> Plants with increased plastidic activity of a starch-phosphorylating enzyme
<130> BCS 04-5008-PCT
<150> EP 04090316.3
   <151> 2001-08-18
<150> US 60/602,454
   <151> 2004-08-18
<160> 4
<170> PatentIn version 3.1
<210> 1
   <211> 3837
   <212> DNA
   <213> Arabidopsis thaliana
<400> 1
   atggcaacct ctaaatccca acaattccag ctaattgaag ggatggagct tcagatcacc   60
   gtcactggat tgccgaatgg gagcagtgta agagctgagt ttcacctgaa gaactgcact  120
   cgcgcatgga ttcttcattg gggttgtatt taccaaggaa ataaccattg gtatatccca  180
   tctgaacatt cctcgaagca aggtgcattg cagactacct ttgtgaagag tggggatgca  240
   tatgtagtga tccttgagtt gcgggatcca agggtacgcg caattgaatt tgttttgaaa  300
   gatggtagcc ataacaggtg gttgagacag cataatggaa acttccgtgt tgagattccc  360
   tggaatgatc tccatgctca tcatcggata ccgaagactc tgatagaaag aagagcacat  420
   aagatatggg accggaaggg acgaccacaa agctctgcac gtgaacaaca gatagactat  480
   gacaatgcag taagagagct ccacgccgag ctcgctagag gaatatcttt agacgaactt  540
   caggctaact ctacagtacc agtggagaaa gaagtaacca gtgagccaca tcacacaatg  600
   atccaatcat atcgccggag gcatgatgtt cagaaatggt tacagaaata tacggaacca  660
   atcaacagaa gtggaagtgt gaaaagttca gcccttgcag aactctccaa gagatctgtg 720
   ggccaagaaa atctagtttc acagaaaagc tttcatgtca gaaactacga gatcacggtc 780
   ctccaaaggg atgttaaggg agattgtcgc ttatggattg ccacgaacat ggcaggtcca 840
   acagttctcc attggggagt cgcaaagtca tctgcaggag agtggttgat accaccacct 900
   gacgtgttac ctgagaaatc aaaatttgtt cacggagcat gtcaaacaca atttaccgat 960
   atgtcgagca gagaacactc ttatcagttt attgatataa atttaaaacg aggtggtttt 1020
   gttggtatcc aatttgtaat atggtcggga ggctactggg tgaacaataa cggagccaac 1080
   ttcgttgtaa acctgaagtc agcagacagt accagtggta agcttgacgt ggatgaaaag 1140
   tatgttctca aatggttact tgatgaaata tctgagcgag agaaagaggc tgagagatca 1200
   ttgatgcaca ggttcaacat tgcaacagag ttgaccgagc gttgtaagga tgaaggagaa 1260
   ggtggatgta ttggtataat ggtgtggatg agattcatgg ccaccagaca tctcacctgg 1320
   aataagaact ataacgtgaa acctcgggaa attagtgaag cactagaaag attcaccaat 1380
   ttgatggaga aaatatattt gcagcaacca aataagagag aaattgtgag actaactatg 1440
   gcacttgtgg gtcgtggagg tcaaggtgat gttgggcaga gaatccgtga cgaaatcctt 1500
   gttattcaga gacataacca ctgcaaatcc ggcatgatgg aagagtggca ccaaaagttg 1560
   cacaacaaca gtagcgcaga tgatgtgata atttgtgagg ctctcttgaa ctatgtgaga 1620
   tctgacttca ggatcgatgc atactggcaa acactacaga ccaatggtct cacaaaagaa 1680
   aggcttgcaa gttatgaccg ccccatagta tcagagcctc gtttcagaag tgattctaaa 1740
   gaaggactca tccgtgacct tacaatgtac ttgaaaacat taaaggcagt tcattcaggt 1800
   gcagaccttg agtctgctat tgatacgttc ctttctccat ctaagggtca tcatgtcttt 1860
   gctgtcaatg gtttatcacc aaaattgcag gatctactga atttagttaa gaggcttgtt 1920
   cgcgaagaga acactgagcc cctaatagag aagttagttg atgctcgcat tcagttacac 1980
   cctgcactac gagcacctcg cacgagggca aaagatttac tatttttgga cattgcgttg 2040
   gagtcatgtt ttaaaacaac aatagagaag agactcatct ctttaaactt caataaccca 2100
   ccggaaatta tatatgtcat ctgcgtggtg cttgagaatc tgtgcttatc tatagttaac 2160
   aatgaagaaa tcatattctg tacaaaggat tggtaccgtg tcagcgaggc ttacagacct 2220
   catgatgttc agtgggcatt gcaaacaaaa gcggtccttg accgtttaca actagtactt 2280
   gctgatagat gtcagcatta ttttacaata atacagccta ctacaaaata tcttggtcaa 2340
   ctgttacgtg ttgacaagca tgggattgat gttttcactg aagaggttat aagagcaggg 2400
   ccaggagccg ttttatcaac tcttgtaaac agatttgatc cttctctaag gaaaatcgcg 2460
   aatttaggct gttggcaggt tatcagcctg gctgatgcat atgggtttgt ggtttgtgtg 2520
   aatgagttaa ttgttgttca gaacaaattc tactcgaagc caactgtaat tattgcaagt 2580
   aaagtcacag gagaagagga gatccctgct ggtgttgtag ccgtgctgac tcctagtatg 2640
   attgatgtct tgtctcatgt atctattaga gcaagaaaca gcaagatatg ctttgctaca 2700
   tgcttcgatc agaatgttct cagcaatctg aagtcgaagg aaggaagagc aatatctatt 2760
   catacaaagt ctactggttt agttatcagt gatggcaaca actctggtgt ctctgttcgt 2820
   catattttta tttcctctgt tccacgggga gtgatctcta aggggaagaa gttttgtggc 2880
   cactatgtga tttcatctaa ggaattcaca gatgaaaggg ttggctcaaa atcatacaat 2940
   ataaaatttc tacgtgaaag agttccatca tggatcaaga tacctacctc agctgctctt 3000
   ccattcggaa catttgagaa tatactctca gatgattcca ataaggatgt agcacgcagg 3060
   atttctgtcc ttaaagattc tcttaacaga ggagacctga caaaacttaa gtccattcaa 3120
   gaagctatct tacaaatgag tgctccaatg gctctgagaa atgaactgat cacaaaattg 3180
   agaagtgaaa gaatgcctta tcttggtgat gaatcaggct ggaaccgctc ctgggtggca 3240
   attaagaagg tctgggcttc aaagtggaac gagagagctt atgtcagttg caaaaaaaat 3300
   aagcttgatc atgatgcggt ctgcatggct gtgctgattc aagaagtcat ttgtggcgat 3360
   tatgctttcg tcattcacac aaacaatccg gtctctggtg actcttcaga aatatacaca 3420
   gagattgtga agggtttggg agagaccttg gttggagcat atccaggacg agcaatgagc 3480
   ttcatcacca agaaaacaaa cctcaagtct ccaaccgtga tcagttaccc aagtaagagg 3540
   ataggtctgt attctaaacc ctcaatcata ttcagatcag attcaaacaa cgaggatctc 3600
   gaaggcaacg caggcgttgg actttacgac agtgtgataa tggatgaagc agaggaagta 3660
   gtggtggatt actcaaggga gccactaata atggacaaat cctttcgagt gcgtctattc 3720
   tcagcgattg cagaagctgg aaatgtgata gagtcaatct atggttgtcc tcaagacatt 3780
   gaaggtgttg tcaaaggtgg acatatctac atcgtccaag ctagacccca agtttaa 3837
<210> 2
   <211> 3987
   <212> DNA
   <213> Artificial Sequence
<220>
   <223> Sequence containing the coding region for the plastidic signal se quence of the oryza sativa dul-I gene fused to the cDNA coding fo r the Arabidopsis tahliana R3 gene
<220>
   <221> CDS
   <222> (1)..(3984)
   <223> coding sequence of the artificial sequence
<220>
   <221> sig_peptide
   <222> (1)..(147)
   <223> coding sequence for the plastidic signal peptide from the Oryza s ativa dul I gene
<220>
   <221> mat_peptide
   <222> (154)..()
   <223> coding sequence of the mature peptide of the R3 (GWD-3) gene from Arabidopsis thaliana missing the natural start codon methionine
<400> 2
   atg gag atg gct ctc cgg cct caa agc ctt cta tgc cct cgg agc agg      48
   Met Glu Met Ala Leu Arg Pro Gln Ser Leu Leu Cys Pro Arg Ser Arg
       -50                 -45                 -40
   ctg aag gtc gtc atc cgg cca gct agc agt gcc agc ggt ggt ggc ctc      96
   Leu Lys Val Val Ile Arg Pro Ala Ser Ser Ala Ser Gly Gly Gly Leu
   -35                 -30                 -25                 -20
   gcg cag tat ttc tta atg acc agg aga tat act gga agc aga att gtt     144
   Ala Gln Tyr Phe Leu Met Thr Arg Arg Tyr Thr Gly Ser Arg Ile Val
                   -15                 -10                 -5
   cga aac ctg gca acc tct aaa tcc caa caa ttc cag cta att gaa ggg     192
   Arg Asn Leu Ala Thr Ser Lys Ser Gln Gln Phe Gln Leu Ile Glu Gly
           -1  1               5                   10
   atg gag ctt cag atc acc gtc act gga ttg ccg aat ggg agc agt gta     240
   Met Glu Leu Gln Ile Thr Val Thr Gly Leu Pro Asn Gly Ser Ser Val
       15                  20                  25
   aga gct gag ttt cac ctg aag aac tgc act cgc gca tgg att ctt cat     288
   Arg Ala Glu Phe His Leu Lys Asn Cys Thr Arg Ala Trp Ile Leu His
   30                  35                  40                  45
   tgg ggt tgt att tac caa gga aat aac cat tgg tat atc cca tct gaa     336
   Trp Gly Cys Ile Tyr Gln Gly Asn Asn His Trp Tyr Ile Pro Ser Glu
                   50                  55                  60
   cat tcc tcg aag caa ggt gca ttg cag act acc ttt gtg aag agt ggg     384
   His Ser Ser Lys Gln Gly Ala Leu Gln Thr Thr Phe Val Lys Ser Gly
               65                  70                  75
   gat gca tat gta gtg atc ctt gag ttg cgg gat cca agg gta cgc gca     432
   Asp Ala Tyr Val Val Ile Leu Glu Leu Arg Asp Pro Arg Val Arg Ala
           80                  85                  90
   att gaa ttt gtt ttg aaa gat ggt agc cat aac agg tgg ttg aga cag     480
   Ile Glu Phe Val Leu Lys Asp Gly Ser His Asn Arg Trp Leu Arg Gln
       95                  100                 105
   cat aat gga aac ttc cgt gtt gag att ccc tgg aat gat ctc cat gct     528
   His Asn Gly Asn Phe Arg Val Glu Ile Pro Trp Asn Asp Leu His Ala
   110                 115                 120                 125
   cat cat cgg ata ccg aag act ctg ata gaa aga aga gca cat aag ata     576
   His His Arg Ile Pro Lys Thr Leu Ile Glu Arg Arg Ala His Lys Ile
                   130                 135                 140
   tgg gac cgg aag gga cga cca caa agc tct gca cgt gaa caa cag ata     624
   Trp Asp Arg Lys Gly Arg Pro Gln Ser Ser Ala Arg Glu Gln Gln Ile
                   145                 150                 155
   gac tat gac aat gca gta aga gag ctc cac gcc gag ctc gct aga gga      672
   Asp Tyr Asp Asn Ala Val Arg Glu Leu His Ala Glu Leu Ala Arg Gly
           160                 165                 170
   ata tct tta gac gaa ctt cag gct aac tct aca gta cca gtg gag aaa      720
   Ile Ser Leu Asp Glu Leu Gln Ala Asn Ser Thr Val Pro Val Glu Lys
       175                 180                 185
   gaa gta acc agt gag cca cat cac aca atg atc caa tca tat cgc cgg      768
   Glu Val Thr Ser Glu Pro His His Thr Met Ile Gln Ser Tyr Arg Arg
   190                 195                 200                 205
   agg cat gat gtt cag aaa tgg tta cag aaa tat acg gaa cca atc aac      816
   Arg His Asp Val Gln Lys Trp Leu Gln Lys Tyr Thr Glu Pro Ile Asn
                   210                 215                 220
   aga agt gga agt gtg aaa agt tca gcc ctt gca gaa ctc tcc aag aga      864
   Arg Ser Gly Ser Val Lys Ser Ser Ala Leu Ala Glu Leu Ser Lys Arg
               225                 230                 235
   tct gtg ggc caa gaa aat cta gtt tca cag aaa agc ttt cat gtc aga      912
   Ser Val Gly Gln Glu Asn Leu Val Ser Gln Lys Ser Phe His Val Arg
           240                 245                 250
   aac tac gag atc acg gtc ctc caa agg gat gtt aag gga gat tgt cgc      960
   Asn Tyr Glu Ile Thr Val Leu Gln Arg Asp Val Lys Gly Asp Cys Arg
       255                 260                 265
   tta tgg att gcc acg aac atg gca ggt cca aca gtt ctc cat tgg gga     1008
   Leu Trp Ile Ala Thr Asn Met Ala Gly Pro Thr Val Leu His Trp Gly
   270                 275                 280                 285
   gtc gca aag tca tct gca gga gag tgg ttg ata cca cca cct gac gtg     1056
   Val Ala Lys Ser Ser Ala Gly Glu Trp Leu Ile Pro Pro Pro Asp Val
                   290                 295                 300
   tta cct gag aaa tca aaa ttt gtt cac gga gca tgt caa aca caa ttt     1104
   Leu Pro Glu Lys Ser Lys Phe Val His Gly Ala Cys Gln Thr Gln Phe
               305                 310                 315
   acc gat atg tcg agc aga gaa cac tct tat cag ttt att gat ata aat     1152
   Thr Asp Met Ser Ser Arg Glu His Ser Tyr Gln Phe Ile Asp Ile Asn
           320                 325                 330
   tta aaa cga ggt ggt ttt gtt ggt atc caa ttt gta ata tgg tcg gga     1200
   Leu Lys Arg Gly Gly Phe Val Gly Ile Gln Phe Val Ile Trp Ser Gly
       335                 340                 345
   ggc tac tgg gtg aac aat aac gga gcc aac ttc gtt gta aac ctg aag     1248
   Gly Tyr Trp Val Asn Asn Asn Gly Ala Asn Phe Val Val Asn Leu Lys
   350                 355                 360                 365
   tca gca gac agt acc agt ggt aag ctt gac gtg gat gaa aag tat gtt     1296
   Ser Ala Asp Ser Thr Ser Gly Lys Leu Asp Val Asp Glu Lys Tyr Val
                   370                 375                 380
   ctc aaa tgg tta ctt gat gaa ata tct gag cga gag aaa gag gct gag     1344
   Leu Lys Trp Leu Leu Asp Glu Ile Ser Glu Arg Glu Lys Glu Ala Glu
               385                 390                 395
   aga tca ttg atg cac agg ttc aac att gca aca gag ttg acc gag cgt     1392
   Arg Ser Leu Met His Arg Phe Asn Ile Ala Thr Glu Leu Thr Glu Arg
           400                 405                 410
   tgt aag gat gaa gga gaa ggt gga tgt att ggt ata atg gtg tgg atg     1440
   Cys Lys Asp Glu Gly Glu Gly Gly Cys Ile Gly Ile Met Val Trp Met
       415                 420                 425
   aga ttc atg gcc acc aga cat ctc acc tgg aat aag aac tat aac gtg    1488
   Arg Phe Met Ala Thr Arg His Leu Thr Trp Asn Lys Asn Tyr Asn Val
   430                 435                 440                 445
   aaa cct cgg gaa att agt gaa gca cta gaa aga ttc acc aat ttg atg    1536
   Lys Pro Arg Glu Ile Ser Glu Ala Leu Glu Arg Phe Thr Asn Leu Met
                   450                 455                 460
   gag aaa ata tat ttg cag caa cca aat aag aga gaa att gtg aga cta    1584
   Glu Lys Ile Tyr Leu Gln Gln Pro Asn Lys Arg Glu Ile Val Arg Leu
               465                 470                 475
   act atg gca ctt gtg ggt cgt gga ggt caa ggt gat gtt ggg cag aga    1632
   Thr Met Ala Leu Val Gly Arg Gly Gly Gln Gly Asp Val Gly Gln Arg
           480                 485                 490
   atc cgt gac gaa atc ctt gtt att cag aga cat aac cac tgc aaa tcc    1680
   Ile Arg Asp Glu Ile Leu Val Ile Gln Arg His Asn His Cys Lys Ser
       495                 500                 505
   ggc atg atg gaa gag tgg cac caa aag ttg cac aac aac agt agc gca    1728
   Gly Met Met Glu Glu Trp His Gln Lys Leu His Asn Asn Ser Ser Ala
   510                 515                 520                 525
   gat gat gtg ata att tgt gag gct ctc ttg aac tat gtg aga tct gac    1776
   Asp Asp Val Ile Ile Cys Glu Ala Leu Leu Asn Tyr Val Arg Ser Asp
                   530                 535                 540
   ttc agg atc gat gca tac tgg caa aca cta cag acc aat ggt ctc aca    1824
   Phe Arg Ile Asp Ala Tyr Trp Gln Thr Leu Gln Thr Asn Gly Leu Thr
               545                 550                 555
   aaa gaa agg ctt gca agt tat gac cgc ccc ata gta tca gag cct cgt    1872
   Lys Glu Arg Leu Ala Ser Tyr Asp Arg Pro Ile Val Ser Glu Pro Arg
           560                 565                 570
   ttc aga agt gat tct aaa gaa gga ctc atc cgt gac ctt aca atg tac    1920
   Phe Arg Ser Asp Ser Lys Glu Gly Leu Ile Arg Asp Leu Thr Met Tyr
       575                 580                 585
   ttg aaa aca tta aag gca gtt cat tca ggt gca gac ctt gag tct gct    1968
   Leu Lys Thr Leu Lys Ala Val His Ser Gly Ala Asp Leu Glu Ser Ala
   590                 595                 600                 605
   att gat acg ttc ctt tct cca tct aag ggt cat cat gtc ttt gct gtc    2016
   Ile Asp Thr Phe Leu Ser Pro Ser Lys Gly His His Val Phe Ala Val
                   610                 615                 620
   aat ggt tta tca cca aaa ttg cag gat cta ctg aat tta gtt aag agg    2064
   Asn Gly Leu Ser Pro Lys Leu Gln Asp Leu Leu Asn Leu Val Lys Arg
               625                 630                 635
   ctt gtt cgc gaa gag aac act gag ccc cta ata gag aag tta gtt gat    2112
   Leu Val Arg Glu Glu Asn Thr Glu Pro Leu Ile Glu Lys Leu Val Asp
           640                 645                 650
   gct cgc att cag tta cac cct gca cta cga gca cct cgc acg agg gca    2160
   Ala Arg Ile Gln Leu His Pro Ala Leu Arg Ala Pro Arg Thr Arg Ala
       655                 660                 665
   aaa gat tta cta ttt ttg gac att gcg ttg gag tca tgt ttt aaa aca    2208
   Lys Asp Leu Leu Phe Leu Asp Ile Ala Leu Glu Ser Cys Phe Lys Thr
   670                 675                 680                 685
   aca ata gag aag aga ctc atc tct tta aac ttc aat aac cca ccg gaa    2256
   Thr Ile Glu Lys Arg Leu Ile Ser Leu Asn Phe Asn Asn Pro Pro Glu
                   690                 695                 700
   att ata tat gtc atc tgc gtg gtg ctt gag aat ctg tgc tta tct ata      2304
   Ile Ile Tyr Val Ile Cys Val Val Leu Glu Asn Leu Cys Leu Ser Ile
               705                 710                 715
   gtt aac aat gaa gaa atc ata ttc tgt aca aag gat tgg tac cgt gtc      2352
   Val Asn Asn Glu Glu Ile Ile Phe Cys Thr Lys Asp Trp Tyr Arg Val
           720                 725                 730
   agc gag gct tac aga cct cat gat gtt cag tgg gca ttg caa aca aaa      2400
   Ser Glu Ala Tyr Arg Pro His Asp Val Gln Trp Ala Leu Gln Thr Lys
       735                 740                 745
   gcg gtc ctt gac cgt tta caa cta gta ctt gct gat aga tgt cag cat      2448
   Ala Val Leu Asp Arg Leu Gln Leu Val Leu Ala Asp Arg Cys Gln His
   750                 755                 760                 765
   tat ttt aca ata ata cag cct act aca aaa tat ctt ggt caa ctg tta      2496
   Tyr Phe Thr Ile Ile Gln Pro Thr Thr Lys Tyr Leu Gly Gln Leu Leu
                   770                 775                 780
   cgt gtt gac aag cat ggg att gat gtt ttc act gaa gag gtt ata aga      2544
   Arg Val Asp Lys His Gly Ile Asp Val Phe Thr Glu Glu Val Ile Arg
               785                 790                 795
   gca ggg cca gga gcc gtt tta tca act ctt gta aac aga ttt gat cct      2592
   Ala Gly Pro Gly Ala Val Leu Ser Thr Leu Val Asn Arg Phe Asp Pro
           800                 805                 810
   tct cta agg aaa atc gcg aat tta ggc tgt tgg cag gtt atc agc ctg      2640
   Ser Leu Arg Lys Ile Ala Asn Leu Gly Cys Trp Gln Val Ile Ser Leu
       815                 820                 825
   gct gat gca tat ggg ttt gtg gtt tgt gtg aat gag tta att gtt gtt      2688
   Ala Asp Ala Tyr Gly Phe Val Val Cys Val Asn Glu Leu Ile Val Val
   830                 835                 840                 845
   cag aac aaa ttc tac tcg aag cca act gta att att gca agt aaa gtc      2736
   Gln Asn Lys Phe Tyr Ser Lys Pro Thr Val Ile Ile Ala Ser Lys Val
                   850                 855                 860
   aca gga gaa gag gag atc cct gct ggt gtt gta gcc gtg ctg act cct      2784
   Thr Gly Glu Glu Glu Ile Pro Ala Gly Val Val Ala Val Leu Thr Pro
               865                 870                 875
   agt atg att gat gtc ttg tct cat gta tct att aga gca aga aac agc      2832
   Ser Met Ile Asp Val Leu Ser His Val Ser Ile Arg Ala Arg Asn Ser
           880                 885                 890
   aag ata tgc ttt gct aca tgc ttc gat cag aat gtt ctc agc aat ctg      2880
   Lys Ile Cys Phe Ala Thr Cys Phe Asp Gln Asn Val Leu Ser Asn Leu
       895                 900                 905
   aag tcg aag gaa gga aga gca ata tct att cat aca aag tct act ggt      2928
   Lys Ser Lys Glu Gly Arg Ala Ile Ser Ile His Thr Lys Ser Thr Gly
   910                 915                 920                 925
   tta gtt atc agt gat ggc aac aac tct ggt gtc tct gtt cgt cat att      2976
   Leu Val Ile Ser Asp Gly Asn Asn Ser Gly Val Ser Val Arg His Ile
                   930                 935                 940
   ttt att tcc tct gtt cca cgg gga gtg atc tct aag ggg aag aag ttt      3024
   Phe Ile Ser Ser Val Pro Arg Gly Val Ile Ser Lys Gly Lys Lys Phe
               945                 950                 955
   tgt ggc cac tat gtg att tca tct aag gaa ttc aca gat gaa agg gtt      3072
   Cys Gly His Tyr Val Ile Ser Ser Lys Glu Phe Thr Asp Glu Arg Val
           960                 965                 970
   ggc tca aaa tca tac aat ata aaa ttt cta cgt gaa aga gtt cca tca      3120
   Gly Ser Lys Ser Tyr Asn Ile Lys Phe Leu Arg Glu Arg Val Pro Ser
       975                 980                 985
   tgg atc aag ata cct acc tca gct gct ctt cca ttc gga aca ttt gag      3168
   Trp Ile Lys Ile Pro Thr Ser Ala Ala Leu Pro Phe Gly Thr Phe Glu
   990                 995                 1000                1005
   aat ata ctc tca gat gat tcc aat aag gat gta gca cgc agg att          3213
   Asn Ile Leu Ser Asp Asp Ser Asn Lys Asp Val Ala Arg Arg Ile
                   1010                1015                1020
   tct gtc ctt aaa gat tct ctt aac aga gga gac ctg aca aaa ctt          3258
   Ser Val Leu Lys Asp Ser Leu Asn Arg Gly Asp Leu Thr Lys Leu
                   1025                1030                1035
   aag tcc att caa gaa gct atc tta caa atg agt gct cca atg gct          3303
   Lys Ser Ile Gln Glu Ala Ile Leu Gln Met Ser Ala Pro Met Ala
                   1040                1045                1050
   ctg aga aat gaa ctg atc aca aaa ttg aga agt gaa aga atg cct          3348
   Leu Arg Asn Glu Leu Ile Thr Lys Leu Arg Ser Glu Arg Met Pro
                   1055                1060                1065
   tat ctt ggt gat gaa tca ggc tgg aac cgc tcc tgg gtg gca att          3393
   Tyr Leu Gly Asp Glu Ser Gly Trp Asn Arg Ser Trp Val Ala Ile
                   1070                1075                1080
   aag aag gtc tgg gct tca aag tgg aac gag aga gct tat gtc agt          3438
   Lys Lys Val Trp Ala Ser Lys Trp Asn Glu Arg Ala Tyr Val Ser
                   1085                1090                1095
   tgc aaa aaa aat aag ctt gat cat gat gcg gtc tgc atg gct gtg          3483
   Cys Lys Lys Asn Lys Leu Asp His Asp Ala Val Cys Met Ala Val
                   1100                1105                1110
   ctg att caa gaa gtc att tgt ggc gat tat gct ttc gtc att cac          3528
   Leu Ile Gln Glu Val Ile Cys Gly Asp Tyr Ala Phe Val Ile His
                   1115                1120                1125
   aca aac aat ccg gtc tct ggt gac tct tca gaa ata tac aca gag          3573
   Thr Asn Asn Pro Val Ser Gly Asp Ser Ser Glu Ile Tyr Thr Glu
                   1130                1135                1140
   att gtg aag ggt ttg gga gag acc ttg gtt gga gca tat cca gga          3618
   Ile Val Lys Gly Leu Gly Glu Thr Leu Val Gly Ala Tyr Pro Gly
                   1145                1150                1155
   cga gca atg agc ttc atc acc aag aaa aca aac ctc aag tct cca          3663
   Arg Ala Met Ser Phe Ile Thr Lys Lys Thr Asn Leu Lys Ser Pro
                   1160                1165                1170
   acc gtg atc agt tac cca agt aag agg ata ggt ctg tat tct aaa          3708
   Thr Val Ile Ser Tyr Pro Ser Lys Arg Ile Gly Leu Tyr Ser Lys
                   1175                1180                1185
   ccc tca atc ata ttc aga tca gat tca aac aac gag gat ctc gaa          3753
   Pro Ser Ile Ile Phe Arg Ser Asp Ser Asn Asn Glu Asp Leu Glu
                   1190                1195                1200
   ggc aac gca ggc gtt gga ctt tac gac agt gtg ata atg gat gaa          3798
   Gly Asn Ala Gly Val Gly Leu Tyr Asp Ser Val Ile Met Asp Glu
                   1205                1210                1215
   gca gag gaa gta gtg gtg gat tac tca agg gag cca cta ata atg          3843
   Ala Glu Glu Val Val Val Asp Tyr Ser Arg Glu Pro Leu Ile Met
                   1220                1225                1230
   gac aaa tcc ttt cga gtg cgt cta ttc tca gcg att gca gaa gct          3888
   Asp Lys Ser Phe Arg Val Arg Leu Phe Ser Ala Ile Ala Glu Ala
                           1235                 1240                 1245
   gga aat gtg ata gag  tca atc tat ggt tgt  cct caa gac att gaa       3933
   Gly Asn Val Ile Glu  Ser Ile Tyr Gly Cys  Pro Gln Asp Ile Glu
                   1250                 1255                 1260
   ggt gtt gtc aaa ggt  gga cat atc tac atc  gtc caa gct aga ccc       3978
   Gly Val Val Lys Gly  Gly His Ile Tyr Ile  Val Gln Ala Arg Pro
                   1265                 1270                 1275
   caa gtt taa                                                         3987
   Gln Val
<210> 3
   <211> 1328
   <212> PRT
   <213> Artificial Sequence
<220>
   <223> Sequence containing the coding region for the plastidic signal se quence of the Oryza sativa dul-I gene fused to the CDNA coding fo r the Arabidopsis tahliana R3 gene
   <400> 3
<210> 4
   <211> 12
   <212> PRT
   <213> Arabidopsis thaliana
<400> 4
   Ile Asp Val Leu Ser His Val Ser Ile Arg Ala Arg
   1 5 10

## Description

The present invention relates to plant cells and plants which are genetically modified, the genetic modification leading to the increase of the activity of a starch-phosphorylating R3 protein in comparison with corresponding non-genetically-modified wild-type plant cells or wild-type plants. Furthermore, the present invention relates to compositions and methods for the generation of such plant cells and plants. Such plant cells and plants synthesize a modified starch. Also described is the starch synthesized by the plant cells and plants according to the invention, methods for the production of this starch, and the production of starch derivatives of this modified starch, and flours comprising this starch.

Moreover, the present invention relates to nucleic acids coding for starch-phosphorylating R3 proteins and to vectors, host cells, plant cells and plants comprising such nucleic acid molecules. Furthermore, the present invention relates to R3 proteins having a starch-phosphorylating activity.

In view of the increasing importance which is currently attached to plant constituents as renewable raw materials, one of the tasks of biotechnological research is to attempt an adaptation of these vegetable raw materials to the requirements of the processing industry. In order to make possible the use of renewable raw materials in as many fields of application as possible, it is additionally necessary to arrive at very diverse substances.

The polysaccharide starch is made of chemically uniform units, the glucose molecules, but constitutes a complex mixture of different forms of molecules which differ with regard to their degree of polymerization and branching and thus differ greatly with regard to their physico-chemical properties. One distinguishes between amylose starch, an essentially unbranched polymer of alpha-1,4-glycosidically linked glucose units, and amylopectin starch, a branched polymer where the branched points are generated by the occurrence of additional alpha-1,6-glycosidic linkages. A further essential difference between amylose and amylopectin is the molecular weight. While amylose, depending on the origin of the starch, has a molecular weight of 5×10⁵ - 10⁶ Da, the molecular weight of amylopectin is between 10⁷ and 10⁸ Da. The two macromolecules can be distinguished on the basis of their molecular weight and their different physico-chemical properties, and the simplest way of visualizing this is based on their different iodine-binding properties.

Amylose has long been regarded as a linear polymer consisting of alpha-1,4-glycosidically linked alpha-D-glucose monomers. More recent studies, however, have revealed the presence of alpha-1,6-glycosidic branched points (approx. 0.1 %) (Hizukuri and Takagi, Carbohydr. Res. 134, (1984), 1-10; Takeda et al., Carbohydr. Res. 132, (1984), 83-92).

The functional properties, such as, for example, solubility, retrogradation behavior, the water-binding capacity, the film-forming properties, viscosity, the gelatinization properties, freeze-thaw stability, the gel strength, the starch granule size of starches are, inter alia, influenced by the amylose/amylopectin ratio, the molecular weight, the side-chain distribution pattern, the ionic content, the lipid and protein content, the mean starch granule size, the starch granule morphology and the like. The functional properties of starch are also influenced in particular by the phosphate content, a non-carbohydrate component of starch. Here, one distinguishes between phosphate which is bonded covalently to the glucose molecules of the starch in the form of monoesters (hereinbelow referred to as starch phosphate) and phosphate in the form of starch-associated phospholipids.

The starch phosphate content varies as a function of the plant variety. Thus, for example, certain maize mutants synthesize a starch with an increased starch phosphate content (waxy maize 0.002% and high-amylose maize (0.013%), while traditional maize varieties only contain traces of starch phosphate. Likewise, small amounts of starch phosphate are found in wheat (0.001%), while no starch phosphate has been detected in oats and sorghum. Likewise, less starch phosphate was found in rice mutants (waxy rice 0.003%) than in traditional rice varieties (0.013%). Significant amounts of starch phosphate have been detected in plants which synthesize tuber or root storage starch, such as, for example, tapioca (0.008%), sweet potato (0.011%), arrowroot (0.021 %) or potato (0.089%). The above-quoted percentages for the starch phosphate content are based in each case on the dry weight of the starch and have been determined by Jane et al. (1996, Cereal Foods World 41 (11), 827-832).

Starch phosphate can be present in the form of monoesters at the C-2, C-3 or C-6 position of the polymerized glucose monomers (Takeda and Hizukuri, 1971, Starch/Stärke 23, 267-272). The phosphate distribution of the phosphate in plant-synthesized starch is generally distinguished by the fact that approximately 30% to 40% of the phosphate residues are bonded covalently in the C-3 position and approximately 60% to 70% of the phosphate residues are bonded covalently in the C-6 position of the glucose molecules (Blennow et al., 2000, Int. J. of Biological Macromolecules 27, 211-218). Blennow et al. (2000, Carbohydrate Polymers 41, 163-174) determine a starch phosphate content which is bonded in the C-6 position of the glucose molecules for different starches such as, for example, potato starch (between 7.8 and 33.5 nmol per mg starch, depending on the variety), starch from different *Curcuma* species (between 1.8 and 63 nmol per mg), tapioca starch (2.5 nmol per mg starch), rice starch (1.0 nmol per mg starch), mung bean starch (3.5 nmol per mg starch) and sorghum starch (0.9 nmol per mg starch). No starch phosphate bonded in the C-6-position was detected by these authors in barley starch and in starch from different waxy mutants of maize. To date, no connection has been made between the genotype of a plant and the starch phosphate content (Jane et al., 1996, Cereal Foods World 41 (11), 827-832). It is currently not possible to influence the starch phosphate content in plants by plant breeding methods.

To date, only one protein which mediates the introduction of covalent bonds of phosphate residues to the starch glucose molecules has been described. This protein has the enzymatic activity of an alpha-glucan, water dikinase (GWD, E.C.: 2.7.9.4) (Ritte et al., 2002, PNAS 99, 7166-7171), is frequently referred to as R1 in the scientific literature and is bound to the starch granules of the storage starch in potato tubers (Lorberth et al., 1998, Nature Biotechnology 16, 473-477). Since storage starch is synthesized in plastids (amyloplasts) and the R1 protein is bound to these starch granules, but is encoded by nuclear-localized nucleic acids, the amino acid sequence is provided with a plastidic signal (transit) peptide (Lorberth et al., 1996, Nature Biotechnology 16, 473-477). In the R1-catalyzed reaction, the starting materials alpha-1,4-glucan (starch), adenosintriphosphate (ATP) and water are converted into the products glucan phosphate (starch phosphate), monophosphate and adenosine monophosphate. In this process, the gamma-phosphate residue of the ATP is transferred to water and the beta-phosphate residue of the ATP to the glucan (starch). The beta-phosphate residue is transferred *in vitro* by R1 from ATP to the C-6 and the C-3 positions of the glucose molecules of alpha-1,4-glucans. The ratio of C-6 phosphate to C-3 phosphate which is obtained in the *in-vitro* reaction corresponds to the ratio found in starch isolated from plants (Ritte et al., 2002, PNAS 99, 7166-7171). Since, in potato starch, approximately 70% of the starch phosphate is found in the C-6 position and approximately 30% in the C-3 position of the glucose monomers of the starch, this means that R1 preferentially phosphorylates the C-6 position of the glucose molecules. Moreover, it has been demonstrated for R1, inter alia by using maize amylopectin, that it is capable of phosphorylating alpha-1,4-glucans, which as yet do not comprise any covalently bonded phosphate (Ritte et al., 2002, PNAS 99, 7166-7171), i.e. R1 is capable of introducing phosphate *de novo* into alpha-1,4-glucans.

Nucleic acid sequences and corresponding amino acid sequences encoding an R1 protein have been described from different species such as, for example, potato (WO 97 11188, GenBank Acc.: AY027522, Y09533), wheat (WO 00 77229, US 6,462,256, GenBank Acc.: AAN93923, GenBank Acc.: AR236165), rice (GenBank Acc.: AAR61445, GenBank Acc.: AR400814), maize (GenBank Acc.: AAR61444, GenBank Acc.: AR400813), soybean (GenBank Acc.: AAR61446, GenBank Acc.: AR400815), citrus (GenBank Acc.: AY094062) and *Arabidopsis* (GenBank Acc.: AF312027).

Wheat plants with an increased activity of an R1 protein as the result of overexpression of an R1 gene from potato are described in WO 02 34923. In comparison with corresponding wild-type plants, in which no starch phosphate was detected, these plants synthesize a starch with significant amounts of starch phosphate in the C-6 position of the glucose molecules.

Further proteins which catalyze a reaction which introduce covalently bonded phosphate groups into the starch have not been described to date, nor are enzymes known which preferably introduce phosphate groups into the C-3 position and/or C-2 position of the glucose molecules of starch. This is why, apart from increasing the starch phosphate content in plants, no means are available of influencing, in a specific fashion, the phosphorylation of starch in plants, of modifying the phosphate distribution within the starch synthesized by plants and/or of further increasing the starch phosphate content.

The present invention is therefore based on the object of providing further possibilities of generating modified starches with an increased phosphate content, and to provide plant cells and/or plants which synthesize such a modified starch, and methods and means for generating said plants and/or plant cells.

This object is achieved in the use forms designated in the claims.

Thus, the present invention describes genetically modified plant cells and genetically modified plants, wherein plastids present in said plant cell or said plants comprise an R3 protein.

The present invention also describes genetically modified plant cells and plants, wherein plastids present in said plant cell or said plants show an increased activity of at least one R3 protein in comparison with plastids present in non-genetically-modified wild-type plant cells.

A first aspect of the present invention describes a plant cell or plant, which is genetically modified, where the genetic modification leads to the presence of an R3 protein or to an increase in the activity of at least one R3 protein in plastids which are present in these plant cells or plants in comparison with plastids present in corresponding non-genetically-modified wild-type plant cells or wild-type plants.

The invention relates to a genetically modified plant cell wherein plastids present in said plant cell comprise a protein which transfers a phosphate residue from a nucleoside triphosphate to starch, wherein the genetic modification consists in the introduction of a foreign nucleic acid molecule into the genome of the plant cell, where the foreign nucleic acid molecule is selected from the group consisting of
a) nucleic acid molecules which code for a protein with the amino acid sequence shown in SEQ ID NO 3,
b) nucleic acid molecules which code for a protein which comprises the amino acid sequence which is encoded by the insertion in plasmid DSM 16587 or by the insertion in plasmid DSM 16645;
c) nucleic acid molecules which code for a protein whose sequence has at least 60% identity with the amino acid sequence shown in SEQ ID NO 3;
d) nucleic acid molecules which code for a protein whose sequence has at least 60% identity with the amino acid sequence which is encoded by the insertion in plasmid DSM 16587 or by the insertion in plasmid DSM 16645;
e) nucleic acid molecules which comprise in the nucleotide sequence shown in SEQ ID NO 1 or SEQ ID NO 2 or a complementary sequence;
f) nucleic acid molecules which comprise the nucleotide sequence of the insertion present in plasmid DSM 16587 or in plasmid DSM 16645;
g) nucleic acid molecules which have at least 60% identity with the nucleic acid sequences described in a), b), e) or f);
h) nucleic acid molecules which hybridize, under stringent conditions with at least one strand of the nucleic acid molecules described in a), b), e) or f);
i) nucleic acid molecules whose nucleotide sequence, as the result of the degeneracy of the genetic code, deviates from the sequence of the nucleic acid molecules mentioned in a), b), e) or f); and
j) nucleic acid molecules which constitute fragments, allelic variants and/or derivatives of the nucleic acid molecules mentioned in a), b), c), d), e), f), g), h) or i)
and wherein the foreign nucleic acid molecule is a recombinant nucleic acid molecule comprising additional sequences which are not naturally present in a combination in which they are present in recombinant nucleic acids where the nucleic acid molecule encoding the protein which transfers a phosphate residue from a nucleoside triphosphate to starch is fused to nucleic acid sequences which code for a plastidic signal peptide so that the recombinant nucleic acid molecule codes for a protein which transfers a phosphate residue from a nucleoside triphosphate to starch which additional to its coding amino acid sequence has a plastidic signal sequence.

In the context of the present invention, the term "wild-type plant cell" means plant cells which were used as starting material for the generation of the plant cells according to the invention, i.e. whose genetic information, apart from the genetic modification which has been introduced, corresponds to that of a plant cell according to the invention.

In the context of the present invention, the term "wild-type plant" means plants which were used as starting material for the generation of the plants according to the invention, i.e. whose genetic information, apart from the genetic modification which has been introduced, corresponds to that of a plant according to the invention.

In the context of the present invention, the term "corresponding" means that, upon comparing a plurality of objects, the objects in question which are compared with one another were kept under identical conditions. In the context of the present invention, the term "corresponding" in the context of wild-type plant cell or wild-type plant means that the plant cells or plants which are compared with one another were grown under identical culture conditions and have an identical (culture) age.

In the context of the present invention, the term "plastid" is understood as meaning cell organelles which are found in plant cells only and which are surrounded by a bilayer and have their own genetic system (plastome, transcription apparatus and translation apparatus). Plastids which are preferred in the context of the present invention are chromoplasts and leucoplasts, especially preferably chloroplasts and particularly preferably amyloplasts.

In connection with the present invention, the term "increased activity of at least one R3 protein" means an increase in the expression of endogenous genes which code

for R3 proteins and/or an increase in the amount of R3 protein in the cells and/or an increase in the enzymatic activity of R3 proteins in the cells.

The increase in the expression can be determined for example by measuring the amount of transcripts which code for R3 protein, for example by Northern blot analysis or by RT-PCR. An increase in this case preferably means an increase in the amount of transcripts in comparison with corresponding non-genetically-modified cells by at least 50%, in particular by at least 70%, preferably by at least 85% and especially preferably by at least 100%. An increase in the amount of transcripts which code for R3 protein also means that plants or plant cells which lack detectable amounts of transcripts which code for R3 protein comprise, after genetic modification according to the invention, detectable amounts of transcripts coding for R3 protein.

The increase in the amount of R3 protein which entails increased activity of these proteins in the plant cells in question can be determined for example by immunological methods such as Western blot analysis, ELISA (enzyme-linked immunosorbent assay) or RIA (radioimmunoassay). An increase in this case preferably means an increase in the amount of R3 protein in comparison with corresponding non-genetically modified cells by at least 50%, in particular by at least 70%, preferably by at least 85% and especially preferably by at least 100%. An increase in the amount of R3 protein also means that plants or plant cells which lack detectable amount of an R3 protein comprise, after genetic modification according to the invention, a detectable amount of an R3 protein.

Methods for raising antibodies which react specifically with a certain protein, i.e. which bind specifically to said protein, are known to the skilled worker (see, for example, Lottspeich and Zorbas (Eds.), 1998, Bioanalytik, Spektrum Akad. Verlag, Heidelberg, Berlin, ISBN 3-8274-0041-4). Such antibodies are raised commercially by some companies (for example Eurogentec, Belgium).

The localization of an R3 protein in plastids of genetically modified plants can be detected by means of subcellular fractionation of plant cells, whereby intact plastids can be obtained. Methods for fractionating subcellular compartments are known to the skilled worker and described in the literature (see, for example, Heldt, 1996, "Pflanzenbiochemie", Spektrum Akad. Verl., ISBN 3-8274-0103-8; Plant Biochemistry, 1997, Dey and Harbome Ed., Academic Press, ISBN 0-12-214674-3). The starting material which can be employed for the isolation of plastids is either plant parts (for example leaves) or protoplasts. If plant parts are employed as starting material, the cells are disrupted by the plant parts first being cut into small sections and subsequently comminuted further in a mixer. The resulting cell homogenate is subsequently subjected to centrifugation.

A further method for the preparation of a cell homogenate comprising intact plastids is first to prepare protoplasts which are subsequently disrupted. The disruption takes place by the protoplast suspension being squeezed through a mesh whose mesh size is smaller than the size of the protoplasts. The cell homogenate obtained is subsequently subjected to centrifugation. The last-mentioned method is particularly suitable for the preparation of cell homogenates of cereal plants, which homogenates comprise intact plastids.

The cell homogenate can be purified further by using differential centrifugation or density centrifugation.

In the case of differential centrifugation, the cell homogenates are centrifuged in a medium whose density is lower than that of the cell organelles. The rate of sedimentation of the individual organelles depends mainly on the particle size. The individual organelles are separated by carrying out a plurality of centrifugations one after the other, with increasing speed. After each centrifugation which has been carried out, the organelles in question are found as the sediment.

In the case of equilibrium centrifugation, the organelles are separated according to density in a centrifugation medium which consists of different media of various densities which are layered one over the other. The density of the individual media decreases from bottom to top. The centrifugation is carried out until all particles of the cell homogenate have reached the zone corresponding to their density.

The isolation of intact amyloplasts is described, inter alia, for potato (Wischmann et al., 1999, Plant Physiology 119, 455-462), maize (Echeveria et al., 1988, Plant Physiology 86, 786-792) and wheat (Tetlow et al., 1993, Planta 189, 597-600), pea (Smith et al., 1990, Planta 180, 517-523).

To determine the purity of an organelle preparation, certain lead enzymes, which are specific for the organelles in question, may be measured in individual organelle fractions. Lead enzymes for certain organelles or subcellular compartments are known to the skilled worker and mentioned inter alia in Strasburger, 1999, "Lehrbuch der Botanik" [Textbook of botany], 34th Edition, Spektrum Akad. Verl., ISBN 3-8274-0779-6.
The fraction of the subcellular compartments which comprises plastids can be analyzed for the presence or for an increased activity of an R3 protein. This can be done for example with immunological methods or by detecting the activity in the plastid fraction.

For the purposes of the present invention, the term "R3 protein" is understood as meaning a protein which transfers a phosphate residue from a nucleoside trisphosphate to starch.

Amino acid sequences which code for R3 proteins comprise a phosphohistidine domain. Phosphohistidine domains are described, for example, in Tien-Shin Yu et al. (2001, Plant Cell 13, 1907-1918). The phosphohistidine domain of the *Arabidopsis thaliana* R3 protein is shown in SEQ ID NO 4.

In comparison to an R1 protein, R3 proteins have no plastidic signal peptide in the amino acid sequences which (naturally) code for them, i.e. R3 proteins which are encoded by plant-endogenous nucleic acids (nucleic acids which naturally occur in the genome of the plant) are located outside the plastids.

The catalysis by an R3 protein of a phosphorylation reaction of a P-starch gives rise to a phosphorylated R3 protein as intermediate, in which a phosphate residue of the ATP is bonded covalently to an amino acid of the R3 protein. The intermediate is formed by the autophosphorylation of the R3 protein, i.e. the R3 protein itself catalyzes the reaction which leads to the intermediate. The autophosphorylation preferentially phosphorylates a histidine residue of the amino acid sequence coding for an R3 protein, especially preferentially a histidine residue which is part of a phosphohistidine domain (Tien-Shin Yu et al., 2001, Plant Cell 13, 1907-1918).

Since, apart from R1, no enzymes which phosphorylate starch have been described to date, it has not been possible to date to increase the starch phosphate content in plants with the aid of other enzymes. This is now possible by using a protein according to the invention or a nucleic acid molecule according to the invention for the genetic modification of plants. Providing an R3 protein leads to the possibility of genetically modifying plants in such a way that they synthesize a starch with modified properties.

In the context of the present invention, the term "starch phosphate" is understood as meaning phosphate groups which are bonded covalently to the glucose molecules of starch.

The activity of an R3 protein can be identified for example by incubating an R3 protein *in vitro* using nucleoside triphosphates comprising a labeled phosphate residue (labeled nucleoside triphosphate). Radiolabeled nucleoside triphosphates are preferably used for this purpose.

Labeled phosphate residues which have been incorporated into starch by an R3 protein can be identified for example by separating the labeled starch (for example by centrifugation, ethanol precipitation, filtration, chromatographic methods and the like) from the remainder of the reaction mixture and subsequently detecting the labeled phosphate residues in the starch fraction. In this context, the labeled phosphate residues which are bound in the starch fraction can be identified for example by determining the amount of the radioactivity present in the starch fraction (for example by means of scintillation counter). Possible detection methods for a protein which requires starch as substrate for a phosphorylation reaction are described hereinbelow in the section General Methods, Item 3.

The positions of the carbon atoms (C-2, C-3 or C-6) of the glucose monomers in starch which are preferentially phosphorylated by an R3 protein can be determined for example by analyzing the P-starches which have been phosphorylated by a protein as described in Ritte et al. (2002, PNAS 99, 7166-7171). To this end, starch which has been phosphorylated by a protein is hydrolyzed using acid and subsequently analyzed by means of anion-exchange chromatography.

The starch which is phosphorylated by an R3 protein is preferably analyzed by means of ³¹P- or ³³P-NMR to determine the positions of the carbon atoms (C-2, C-3 or C-6) of the glucose monomers in the P-starch which are phosphorylated. An especially preferred method for identifying the positions of the C atoms of a glucose molecule of a starch which are phosphorylated by an R3-protein-catalyzed reaction is described further below in the section General Methods, Item 4.

The amino acid sequence shown in SEQ ID NO 3 codes for an R3 protein from *Arabidopsis thaliana,* the amino acid sequence shown in SEQ ID NO 3 being in (translational) fusion with a plastidic signal peptide, giving rise to a recombinant amino acid sequence which codes for an R3 protein which, in addition to its coding amino acid sequence, has a plastidic signal peptide.

In a further embodiment of the present invention, amino acid sequences coding for R3 proteins have at least 60%, in particular at least 70%, preferably at least 80% and especially preferably at least 90% and particularly preferably at least 95% identity with the sequence shown in SEQ ID NO 3.

In a further embodiment of the present invention, the R3 protein has a phosphohistidine domain (Tien-Shin Yu et al., 2001, Plant Cell 13, 1907-1918). Amino acid sequences which code for R3 proteins comprising a phosphohistidine domain having at least 85%, in particular at least 88%, preferably at least 91% and especially preferably at least 94% and particularly preferably at least 97% identity with the amino acid sequence of the phosphohistidine domain of the R3 protein from *Arabidopsis thaliana* shown in SEQ ID NO 4.

The present invention describes a genetically modified plant cell according to the invention or to a genetically modified plant according to the invention where the genetic modification consists in the introduction of at least one foreign nucleic acid molecule into the genome of the plant.

In this context, the term "genetic modification" means the introduction of homologous and/or heterologous foreign nucleic acid molecules into the genome of a plant cell or into the genome of a plant, where said introduction of these molecules leads to an increase in the activity of an R3 protein.
Owing to the introduction of a foreign nucleic acid molecule, the plant cells according to the invention or plants according to the invention are modified with regard to their genetic information. The presence or the expression of the foreign nucleic acid molecule leads to a phenotypic change. "Phenotypic" change preferably means a measurable, change in one or more cellular functions. For example, the genetically modified plant cells according to the invention and the genetically modified plants according to the invention show an increase in the activity of an R3 protein as the result of the presence, or upon expression, of the nucleic acid molecule which has been introduced.

In the context of the present invention, the term "foreign nucleic acid molecule" means a molecule which either does not occur naturally in corresponding wild-type plant cells or which does not occur naturally in wild-type plant cells in that specific spatial arrangement or which is localized at a locus in the genome of the wild-type plant cell at which it does not occur naturally. Preferably, the foreign nucleic acid molecule is a recombinant molecule which consists of different elements whose combination or specific spatial arrangement does not occur naturally in plant cells.

In the context of the present invention, the term "genome" is understood as meaning the entirety of the hereditary material present in a plant cell. The skilled worker knows that not only the nucleus, but other compartments too (for example plastids, mitochondria) comprise hereditary material.

In a further embodiment, the plant cells according to the invention and the plants according to the invention comprise the foreign nucleic acid molecule coding for an R3 protein, preferably an *Arabidopsis thaliana* R3 protein.

In a further embodiment, the foreign nucleic acid molecule codes for an R3 protein with the amino acid sequence shown in SEQ ID NO 3.

A multiplicity of techniques is available for introducing DNA into a plant host cell. These techniques comprise the transformation of plant cells with T-DNA using *Agrobacterium tumefaciens* or *Agrobacterium rhizogenes* as transformation agent, the fusion of protoplasts, injection, electroporation of DNA, the introduction of the DNA by means of the biolistic approach, and other possibilities.
The use of the agrobacteria-mediated transformation of plant cells has been studied intensively and has been described sufficiently in EP 120516; Hoekema, IN: The Binary Plant Vector System Offsetdrukkerij Kanters B.V. Alblasserdam (1985), Chapter V; Fraley et al., Crit. Rev. Plant Sci. 4, 1-46 and in An et al. EMBO J. 4, (1985), 277-287. For the transformation of potato, see, for example, Rocha-Sosa et al., EMBO J, 8, (1989), 29-33).

The transformation of monocotyledonous plants by means of vectors based on *Agrobacterium* transformation has also been described (Chan et al., Plant Mol. Biol. 22, (1993), 491-506; Hiei et al., Plant J. 6, (1994) 271-282; Deng et al, Science in China 33, (1990), 28-34; Wilmink et al., Plant Cell Reports 11, (1992), 76-80; May et al., Bio/Technology 13, (1995), 486-492; Conner and Domisse, Int. J. Plant Sci. 153 (1992), 550-555; Ritchie et al, Transgenic Res. 2, (1993), 252-265). An alternative system for the transformation of monocotyledonous plants is the transformation by means of the biolistic approach (Wan and Lemaux, Plant Physiol. 104, (1994), 37-48; Vasil et al., Bio/Technology 11 (1993), 1553-1558; Ritala et al., Plant Mol. Biol. 24, (1994), 317-325; Spencer et al., Theor. Appl. Genet. 79, (1990), 625-631), the transformation of protoplasts, the electroporation of partially permeabilized cells; the introduction of DNA by means of glass fibers. The transformation of maize, in particular, is described repeatedly in the literature (cf., for example, WO95/0612 EP0513849, EP0465875, EP0292435; Fromm et al., Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet 80, (1990), 721-726).

The successful transformation of other cereal species has also been described, for example for barley (Wan and Lemaux, *supra*; Ritala et al., *supra*; Krens et al., Nature 296, (1982), 72-74) and for wheat (Nehra et al., Plant J. 5, (1994), 285-297; Becker et al., 1994, Plant Journal 5, 299-307). All the above methods are suitable for the purposes of the present invention.

Plant cells and plants which are genetically modified by the introduction of an R3 protein can be distinguished from wild-type plant cells, or wild-type plants, inter alia by the fact that they comprise a foreign nucleic acid molecule which does not naturally occur in wild-type plant cells, or wild-type plants, or by the fact that such a molecule is integrated at a locus in the genome of the plant cell according to the invention or in the genome of the plant according to the invention at which it does not occur in wild-type plant cells, or wild-type plants, i.e. in a different genomic environment. Furthermore, such plant cells according to the invention and plants according to the invention can be distinguished from wild-type plant cells and wild-type plants, respectively, by the fact that they comprise stably integrated into their genome at least one copy of the foreign nucleic acid molecule, if appropriate in addition to copies of such a molecule which naturally occur in the wild-type plant cells or wild-type plants. If the foreign nucleic acid molecule(s) introduced into the plant cells according to the invention or plants according to the invention take(s) the form of additional copies to molecules which already naturally occur in the wild-type plant cells or wild-type plants, the plant cells according to the invention and the plants according to the invention can be distinguished from wild-type plant cells and wild-type plants, respectively, by the fact that this additional copy, or these additional copies, is/are localized at loci in the genome at which it does not occur, or they do not occur, in wild-type plant cells or wild-type plants. This can be verified for example with the aid of a Southern blot analysis.
Furthermore, the plant cells according to the invention and the plants according to the invention can be distinguished from wild-type plant cells and wild-type plants, respectively, by preferably at least one of the following traits: if the foreign nucleic acid molecule introduced is heterologous with regard to the plant cell or plant; the plant cells according to the invention or plants according to the invention comprise transcripts of the nucleic acid molecules introduced. This can be verified for example by Northern blot analysis or by RT-PCR (reverse transcription polymerase chain reaction). Plant cells according to the invention and plants according to the invention which express an antisense and/or RNAi transcript can be detected for example with the aid of specific nucleic acid probes which are complementary to the RNA coding for the protein (and which occurs naturally in the plant cell). Preferably, the plant cells according to the invention and the plants according to the invention comprise a protein which is encoded by a nucleic acid molecule which has been introduced. This can be detected for example by immunological methods, in particular by a Western blot analysis.

If the foreign nucleic acid molecule which has been introduced is homologous with regard to the plant cell or plant, the plant cells according to the invention and the plants according to the invention can be distinguished from wild-type plant cells and wild-type plants, respectively, for example on the basis of the additional expression of the foreign nucleic acid molecules which have been introduced. The plant cells according to the invention and the plants according to the invention preferably comprise transcripts of the foreign nucleic acid molecules. This can be detected for example by Northern blot analysis or with the aid of what is known as quantitative PCR.

In a further embodiment, the plant cells according to the invention and plants according to the invention take the form of transgenic plant cells and transgenic plants, respectively.

In a further embodiment, the present invention describes plant cells according to the invention and plants according to the invention where the foreign nucleic acid molecule is selected from the group consisting of
a) nucleic acid molecules which code for a protein with the amino acid sequence shown as SEQ ID NO 3;
b) nucleic acid molecules which code for a protein which comprises the amino acid sequence which is encoded by the insertion in plasmid pIR138-210 or by the insertion in plasmid pIR139-210;
c) nucleic acid molecules which code for a protein whose sequence has at least 60% identity with the amino acid sequence shown in SEQ ID NO 3;
d) nucleic acid molecules which code for a protein whose sequence has at least 60% identity with the amino acid sequence which is encoded by the coding region of the insertion in plasmid pIR138-210 or by the insertion in plasmid pIR139-210;
e) nucleic acid molecules which comprise the nucleotide sequence shown in SEQ ID NO1 or SEQ ID NO 2 or a complementary sequence;
f) nucleic acid molecules which comprise the nucleotide sequence of the insertion present in plasmid pIR138-210 or in plasmid pIR139-210;
g) nucleic acid molecules which have at least 60% identity with the nucleic acid sequences described in a), b), e) or f);
h) nucleic acid molecules which hybridize under stringent conditions with at least one strand of the nucleic acid molecules described in a), b), d), e) or f);
i) nucleic acid molecules whose nucleotide sequence, as the result of the degeneracy of the genetic code, deviates from the sequence of the nucleic acid molecules mentioned in a), b), e) or f); and
j) nucleic acid molecules which constitute fragments, allelic variants and/or derivatives of the nucleic acid molecules mentioned in a), b), c), d), e), f), g), h) or i).

The amino acid sequence shown in SEQ ID NO 3 codes for an R3 protein from *Arabidopsis thaliana.*

The proteins encoded by the different variants of the nucleic acid molecules according to the invention share certain characteristics. These may include for example biological activity, molecular weight, immunological reactivity, conformation and the like, and physical properties such as, for example, the migration behavior in gel electrophoreses, chromatographic behavior, sedimentation coefficients, solubility, spectroscopic properties, stability, pH optimum, temperature optimum and the like.
The molecular weight of the *Arabidopsis thaliana* R3 protein deduced from the amino acid sequence shown in SEQ ID NO 2 is approx. 131 kDa. The molecular weight of a protein according to the invention deduced from the coding amino acid sequence is therefore preferably in the range of from 120 kDa to 145 kDa, preferably from 120 kDa to 140 kDa, especially preferably from 125 kDa to 140 kDa, particularly preferably from 130 kDa to 135 kDa.
The amino acid sequence shown in SEQ ID NO 3 which codes for an *Arabidopsis thaliana* R3 protein comprises a phosphohistidine domain. An R3 protein according to the invention therefore preferably comprises a phosphohistidine domain which has at least 85%, preferably at least 88%, especially preferably at least 91% and particularly preferably at least 95% identity with the phosphohistidine domain shown in SEQ ID NO 4.

The present invention relates to nucleic acid molecules which code for a protein with the enzymatic activity according to the invention of an R3 protein, where the encoded R3 protein has at least 70%, preferably at least 80%, especially preferably at least 90% and particularly preferably at least 95% identity with the amino acid sequences shown in SEQ ID NO 3.

An *Escherichia coli* strain comprising the plasmid pIR138-210 comprising a cDNA which codes for an *Arabidopsis thaliana* R3 protein which is in translational fusion with a nucleic acid sequence coding for the plastidic signal peptide of the *Oryza sativa dul l* gene has been deposited at the Deutsche Sammlung von Mikroorganismen und Zellkulturen GmbH, Mascheroder Weg 1b, 38124 Braunschweig, Germany, in compliance with the provisions of the Budapest Treaty on July 21. 2004, under the number DSM 16587. The amino acid sequence shown in SEQ ID NO 3 can be derived from the coding region of the cDNA sequence integrated in plasmid pIR138-210 and codes for an *Arabidopsis thaliana* R3 protein which has a plastidic signal peptide. The present invention therefore also relates to nucleic acid molecules which code for a protein with the enzymatic activity of an R3 protein which comprises the amino acid sequence encoded by the insertion in plasmid pIR138-210, the encoded protein having at least 70%, preferably at least 80%, especially preferably at least 90% and particularly preferably 95% identity with the amino acid sequence which can be deduced from the insertion in pIR138-210.

The plasmid pIR139-210 which comprises a cDNA encoding for an *Arabidopsis thaliana* R3 protein has been deposited at the Deutsche Sammlung von Mikroorganismen und Zellkulturen GmbH, Mascheroder Weg 1b, 38124 Braunschweig, Germany, in compliance with the provisions of the Budapest Treaty on August 10, 2004, under the number DSM 16645. The present invention therefore also relates to nucleic acid molecules which code for a protein with the enzymatic activity of an R3 protein which comprises the amino acid sequence encoded by the insertion in plasmid pIR139-210, the encoded protein having at least 70%, preferably at least 80%, especially preferably at least 90% and particularly preferably 95% identity with the amino acid sequence which can be deduced from the insertion in pIR139-210.

The nucleic acid sequence shown in SEQ ID NO 1 is a cDNA sequence which comprises the coding region for an *Arabidopsis thaliana* R3 protein.

The present invention therefore also relates to nucleic acid molecules which code for an R3 protein and which comprise the coding region of the nucleotide sequences shown in SEQ ID NO 1 or sequences which are complementary thereto, nucleic acid molecules which comprise the coding region of the nucleotide sequence of the insertion present in plasmid pIR139-210 and nucleic acid molecules which have at least 70%, preferably at least 80%, especially preferably at least 90% and particularly preferably at least 95% identity with the abovementioned nucleic acid molecules.

The nucleic acid sequence shown in SEQ ID NO 2 is a cDNA sequence which comprises the coding region for an *Arabidopsis thaliana* R3 protein which is in translational fusion with the nucleic acid sequence coding for the plastidic signal peptide of the *Oryza sativa dul* / gene.
The present invention also relates to nucleic acid molecules which code for an R3 protein and which comprise the coding region of the nucleotide sequences shown in SEQ ID NO 2 or sequences complementary thereto, to nucleic acid molecules which comprise the coding region of the nucleotide sequence of the insertion present in the plasmid pIR138-210 and to nucleic acid molecules which have at least 70%, preferably at least 80%, especially preferably at least 90% and particularly preferably at least 95% identity with the abovementioned nucleic acid molecules.

The skilled worker can, with the aid of the sequence information of nucleic acid molecules according to the invention, or with the aid of a nucleic acid molecule according to the invention, isolate homologous sequences from other plant species. This can be done for example with the aid of conventional methods, such as screening cDNA libraries or genomic libraries with suitable hybridization probes. The skilled worker knows that homologous sequences can also be isolated with the aid of (degenerate) oligonucleotides and using PCR-based methods.
Screening databases as are provided by, for example, EMBL (http://www.ebi.ac.uk/Tools/index.htm) or NCBI (National Center for Biotechnology Information, hftp://www.ncbi.nlm.nih.govl) can also serve for identifying homologous sequences which code for R3 protein.

Here, one or more sequences is, or are, entered as what is known as a query. This query sequence is then compared with sequences in the selected databases by means of statistic computer programs. Such database queries (for example blast or fasta searches) are known to the skilled worker and can be carried out using various providers.
If such a database query is carried out for example at the NCBI (National Center for Biotechnology Information, hftp://www.ncbi.nlm.nih.gov/), the standard settings, which are specified for the respective comparative search, should be used. For protein sequence comparisons (blastp), these settings are as follows: Limit entrez = not activated; Filter = low compexity activated; Expect value = 10; word size = 3; Matrix = BLOSUM62; Gap costs: Existence =11, Extension = 1.
The following parameters are to be set for nucleic acid sequence comparison (blastn): Limit entrez = not activated; Filter = low compexity activated; Expect value = 10; word size =11.

In the case of such a database search, for example the sequences described in the present invention can be used as query sequence in order to identify further nucleic acid molecules and/or proteins which code for an R3 protein.
Using the above-described methods, it is also possible to identify and/or isolate nucleic acid molecules according to the invention which hybridize with the sequence shown in SEQ ID NO 1 and which code for an R3 protein.
For the purposes of the present invention, the term "hybridization" means hybridization under conventional hybridization conditions, preferably under stringent conditions as they are described for example in Sambrook et al. (Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition (2002), ISBN: 0471250929). Especially preferably, "hybridization" means hybridization under the following conditions:
Hybridization buffer:
   2xSSC; 10xDenhardt solution (Ficoll 400+PEG+BSA; ratio 1:1:1); 0.1% SDS;
   5 mM EDTA; 50 mM Na₂HPO₄; 250 µg/ml herring sperm DNA; 50 µg/ml tRNA; or
   25 M sodium phosphate buffer pH 7.2; 1 mM EDTA; 7% SDS
Hybridization temperature:
   T = 65° to 68°C
   Wash buffer: 0.1xSSC; 0.1% SDS
   Wash temperature: T=65° to 68°C.

In principle, nucleic acid molecules which hybridize with nucleic acid molecules according to the invention can be derived from any plant species. Nucleic acid molecules which hybridize with the molecules according to the invention can be isolated for example from genomic libraries or from cDNA libraries. Such nucleic acid molecules can be identified and isolated using the nucleic acid molecules according to the invention or parts of these molecules or the reverse complements of these molecules, for example by means of hybridization by standard methods (see, for example, Sambrook et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons, 5th edition (2002), ISBN: 0471250929) or by amplification by means of PCR.
Examples of hybridization probes which can be used are nucleic acid molecules with precisely, or essentially, the nucleotide sequence stated in SEQ ID NO 1, or parts of these sequences. The fragments used as hybridization probe can also take the form of synthetic fragments or oligonucleotides which have been generated with the aid of the customary synthetic techniques and whose sequence essentially agrees with the sequence of a nucleic acid molecule according to the invention. Once genes which hybridize with the nucleic acid sequences according to the invention have been identified and isolated, they should be sequenced and the characteristics of the proteins encoded by this sequence should be analyzed to ascertain whether it is an R3 protein. Methods which are particularly suitable are homology comparisons at the nucleic acid or amino acid sequence level and the determination of the enzymatic activity. The activity of an R3 protein can be determined for example as described above in General Methods Item 2.
The molecules which hybridize with the nucleic acid molecules according to the invention comprise in particular fragments, derivatives and allelic variants of the nucleic acid molecules according to the invention which code for an R3 protein from plants, in particular an R3 protein from *Arabidopsis thaliana* plants. In the context of the present invention, the term "derivative" means that the sequences of these molecules differ at one or more positions from the sequences of the above-described nucleic acid molecules and have a high degree of identity with these sequences. The deviations from the above-described nucleic acid molecules may have originated for example as the result of deletion, addition, substitution, insertion or recombination.

In the context of the present invention, the term "identity" means a sequence identity over the entire length of the coding region (full-length sequence) of at least 70%, in particular an identity of at least 80%, preferably over 85%, especially preferably over 90% and in particular at least 95%. In the context of the present invention, the term "identity" is understood as meaning the number of amino acids/nucleotides which agree (identity) with other proteins/nucleic acids, expressed in percent. Preferably, the identity is determined by comparing the SEQ ID NO 3, in the case of amino acids, or SEQ ID NO 1, in the case of nucleic acids, with other proteins/nucleic acids with the aid of computer programs. If full-length sequences which are compared with one another differ with regard to their lengths, the identity is to be determined in such a way that the number of amino acids which the shorter sequence shares with the longer sequence determines the percentage of the identity. The identity is preferably determined by means of the known computer program ClustalW which is available to the public (Thompson et al., Nucleic Acids Research 22 (1994), 4673-4680). ClustalW is made available to the public by Julie Thompson (Thompson@EMBL-Heidelberg.DE) and Toby Gibson (Gibson@EMBL-Heidelberg.DE), European Molecular Biology Laboratory, Meyerhofstrasse 1, D 69117 Heidelberg, Germany. Likewise, ClustalW can be downloaded from different internet pages, inter alia at the IGBMC (Institut de Génétique et de Biologie Moléculaire et Cellulaire, B.P. 163, 67404 Illkirch Cedex, France; ftp://ftp-igbmc.u-strasbg.fr/pub/) and at the EBI (ftp://ftp.ebi.ac.uk/pub/software/) and at all mirrored internet pages of the EBI (European Bioinformatics Institute, Welcome Trust Genome Campus, Hinxton, Cambridge CB10 1SD, UK).
To determine the identity between proteins according to the invention and other proteins, it is preferred to use the ClustalW computer program Version 1.8. The following parameters are to be set: KTUPLE=1, TOPDIAG=5, WINDOW=5, PAIRGAP=3, GAPOPEN=10. GAPEXTEND=0.05, GAPDIST=8, MAXDIV=40, MATRIX=GONNET, ENDGAPS(OFF), NOPGAP, NOHGAP.
To determine the identity between, for example, the nucleotide sequence of the nucleic acid molecules according to the invention and the nucleotide sequence of other nucleic acid molecules, it is preferred to use the ClustalW computer program Version 1.8. The following parameters are to be set:
KTUPLE=2, TOPDIAGS=4, PAIRGAP=5, DNAMATRIX:IUB, GAPOPEN=10, GAPEXT=5, MAXDIV=40, TRANSITIONS: unweighted.

Identity furthermore means that functional and/or structural equivalence exists between the nucleic acid molecules in question or the proteins encoded by them. The nucleic acid molecules which are homologous to the above-described molecules and which are derivatives of these molecules generally take the form of variations of these molecules which are modifications exerting the same biological function. They may take the form of naturally occurring variations, for example sequences from other plant species, or of mutations, it being possible for these mutations to have occurred naturally or to have been introduced by site-directed mutagenesis. Furthermore, the variations may take the form of synthetically generated sequences. The allelic variants can take the form of naturally occurring variants, but also of synthetically generated variants or variants produced by recombinant DNA techniques. A specific form of derivatives are, for example, nucleic acid molecules which deviate from nucleic acid molecules according to the invention as the result of the degeneracy of the genetic code.

The proteins encoded by the different derivatives of the nucleic acid molecules according to the invention share certain characteristics. These may include, for example, biological activity, substrate specificity, molecular weight, immunological reactivity, conformation and the like, and physical properties such as, for example, the migration behavior in gel electrophoreses, chromatographic behavior, sedimentation coefficients, solubility, spectroscopic properties, stability, pH optimum, temperature optimum and the like. Preferred properties of an R3 protein have already been detailed above and apply analogously here.

The nucleic acid molecules according to the invention can be any nucleic acid molecules, in particular DNA or RNA molecules, for example cDNA, genomic DNA, mRNA and the like. They may be naturally occurring molecules or molecules generated by recombinant or chemical synthetic methods. They can be single-stranded molecules which comprise either the coding or the noncoding strand, or double-stranded molecules.

In the context of the present invention, plant cells according to the invention and plants according to the invention can also be generated by using what is known as insertion mutagenesis (review article: Thomeycroft et al., 2001, Journal of experimental Botany 52 (361), 1593-1601). In the context of the present invention, insertion mutagenesis is to be understood as meaning in particular the insertion of transposons or what is known as transfer DNA (T-DNA) into a gene or into the vicinity of a gene encoding an R3 protein, thus increasing the activity of an R3 protein in the cell in question.
In this context, the transposons may take the form of transposons which occur naturally in the cell (endogenous transposons) or those which do not occur naturally in said cell but which have been introduced into the cell by means of recombinant methods, such as, for example, transformation of the cell (heterologous transposons). The skilled worker is familiar with modifying the expression of genes by means of transposons. An overview over the use of endogenous and heterologous transposons as tools in plant biotechnology is given in Ramachandran and Sundaresan (2001, Plant Physiology and Biochemistry 39, 234-252).
T-DNA insertion mutagenesis is based on the ability of certain segments (T-DNA) of Ti-plasmids from Agrobacterium to integrate into the genome of plant cells. The site of integration into the plant chromosome is not fixed but may take place at any location. If the T-DNA integrates into a segment or into the vicinity of a segment of the chromosome which constitutes a gene function, this may lead to an increase in the gene expression, and thus also to a modification of the activity of a protein encoded by the gene in question.
In this context, the sequences inserted into the genome (in particular transposons or T-DNA) are distinguished by the fact that they comprise sequences which lead to an activation of regulatory sequences of an R3 gene ("activation tagging").

Plant cells and plants according to the invention can be generated with the aid of what is known as activation tagging (see, for example, Walden et al., Plant J. (1991), 281-288; Walden et al., Plant Mol. Biol. 26 (1994), 1521-1528). This method is based on the activation of endogenous promoters by enhancer sequences, such as, for example, the enhancer of the 35S RNA promoter of the cauliflower mosaic virus, or the octopine synthase enhancer.

In the context of the present invention, the term "T-DNA activation tagging" is understood as meaning a T-DNA fragment which comprises enhancer sequences and which, as the result of integration into the genome of a plant cell, leads to the increase in the activity of at least one R3 protein.

In the context of the present invention, the term "transposon activation tagging" is understood as meaning a transposon which comprises enhancer sequences and which, as the result of integration into the genome of a plant cell, leads to the increase in the activity of at least one R3 protein.

In a further embodiment, the DNA molecules according to the invention which code for an R3 protein are linked with regulatory sequences which initiate the transcription in plant cells (promoters) and which lead to an increase in an R3 protein activity in the cell. In this context, the nucleic acid molecules according to the invention are in sense orientation relative to the regulatory sequences.

To express nucleic acid molecules which code for an R3 protein, these molecules are preferably linked with regulatory DNA sequences which ensure the transcription in plant cells. These include in particular promoters. In general, any promoter which is active in plant cells is suitable for the purposes of expression.
In this context, the promoter may be chosen in such a way that expression takes place constitutively or only in a particular tissue, at a particular point in time of plant development or at a point in time which is determined by external factors. The promoter can be homologous or heterologous both with regard to the plant and with regard to the nucleic acid molecule.
Examples of suitable promoters are the promoter of the cauliflower mosaic virus 35S RNA and the maize ubiquitine promoter for constitutive expression, the patatin gene promoter B33 (Rocha-Sosa et al., EMBO J. 8 (1989), 23-29) for tuber-specific expression in potatoes, or a promoter which ensures expression in photosynthetically active tissues only, for example the ST-LS1 promoter (Stockhaus et al., Proc. Natl. Acad. Sci. USA 84 (1987), 7943-7947; Stockhaus et al., EMBO J. 8 (1989), 2445-2451) or, for endosperm-specific expression, the wheat HMG promoter, the USP promoter, the phaseolin promoter, promoters of maize zeine genes (Pedersen et al., Cell 29 (1982), 1015-1026; Quatroccio et al., Plant Mol. Biol. 15 (1990), 81-93), glutelin promoter (Leisy et al., Plant Mol. Biol. 14 (1990), 41-50; Zheng et al., Plant J. 4 (1993), 357-366; Yoshihara et al., FEBS Lett. 383 (1996), 213-218) or Shrunken-1 promoter (Werr et al., EMBO J. 4 (1985), 1373-1380). However, it is also possible to use promoters which are activated only at a point in time which is determined by external effects (see, for example, WO 9307279). Promoters which may be of specific interest in this context can be promoters of heat shock proteins, which are simple to induce. Furthermore it is possible to use seed-specific promoters, such as, for example, the Vicia faba USP promoter, which ensures seed-specific expression in Vicia faba and other plants (Fiedler et al., Plant Mol. Biol. 22 (1993), 669-679; Bäumlein et al., Mol. Gen. Genet. 225 (1991),459-467).

Furthermore, a termination sequence (polyadenylation signal), which serves for adding a poly-A tail to the transcript, may be present. The poly-A tail is said to have a function in stabilizing the transcripts. Such elements are described in the literature (cf. Gielen et al., EMBO J. 8 (1989), 23-29) and can be exchanged as desired.

Intron sequences between the promoter and coding region may also be present. Such intron sequences can lead to stable expression and to an increased expression in plants (Callis et al., 1987, Genes Devel. 1, 1183-1200; Luehrsen, and Walbot, 1991, Mol. Gen. Genet. 225, 81-93; Rethmeier, et al., 1997; Plant Journal. 12(4):895-899; Rose and Beliakoff, 2000, Plant Physiol. 122 (2), 535-542; Vasil et al., 1989, Plant Physiol. 91, 1575-1579; XU et al., 2003, Science in China Series C vol. 46 No. 6, 561-569). Examples of suitable intron sequences are the first intron of the maize sh1 gene, the first intron of the maize poly-ubiquitine gene 1, the first intron of the rice EPSPS gene or one of the two first introns of the *Arabidopsis* PAT1 gene. Suitable intron sequences are known to the skilled worker and described extensively in the literature.

Furthermore, plant cells according to the invention and plants according to the invention can be generated by what is known as *"in-situ* activation", where the genetic modification which has been introduced brings about a modification of the regulatory sequences of endogenous R3 genes, which leads to an enhanced expression of R3 genes. The activation of an R3 gene is preferably effected by "*in-vivo*" mutagenesis of a promoter or of enhancer sequences of an endogenous R3 gene. In this context, a promoter or an enhancer sequence may be modified by means of mutagenesis in such a manner that the mutation generated leads, in plant cells according to the invention or plants according to the invention, to an increased expression of an R3 gene in comparison with the expression of an R3 gene in wild-type plant cells or wild-type plants. The mutation in a promoter or enhancer sequence may also lead to R3 genes being expressed, in plant cells according to the invention or plants according to the invention, at a point in time at which they are not expressed in wild-type plant cells or wild-type plants.

In the context of the present invention, the term "R3 gene" is understood as meaning a nucleic acid molecule (cDNA, DNA) which codes for an R3 protein, preferably an *Arabidopsis thaliana* R3 protein.

In the course of what is known as "*in-vivo* mutagenesis", an RNA/DNA hybrid oligonucleotide ("chimeroplast") is introduced into plant cells by transforming plant cells (Kipp, P.B. et al., Poster Session at the "5th International Congress of Plant Molecular Biology, September 21-27 1997, Singapore; R.A. Dixon and C.J. Amtzen, Meeting report on "Metabolic Engineering in Transgenic Plants", Keystone Symposia, Copper Mountain, CO, USA, TIBTECH 15, (1997), 441-447; International patent application WO 9515972; Kren et al., Hepatology 25, (1997), 1462-1468; Cole-Strauss et al., Science 273, (1996), 1386-1389; Beetham et al., 1999, PNAS 96, 8774-8778).
A part of the component of the RNA/DNA oligonucleotide is homologous to a nucleic acid sequence of an endogenous R3 gene, but comprises a mutation in comparison with the nucleic acid sequence of an endogenous R3 gene or comprises a heterologous region which is surrounded by the homologous regions.
As the result of the homologous regions of the RNA/DNA oligonucleotide and of the endogenous nucleic acid molecule undergoing basepairing, followed by homologous recombination, the mutation, or heterologous region, present in the DNA component of the RNA/DNA oligonucleotide can be transferred into the genome of a plant cell. This leads to the increase in the activity of one or more R3 proteins.

All of these methods are based on the introduction of a foreign nucleic acid molecule into the genome of a plant cell or plant and are therefore, in principle, suitable for generating plant cells according to the invention and plants according to the invention.

In a preferred embodiment of the present invention, the foreign nucleic acid molecules according to the invention take the form of recombinant nucleic acid molecules wherein nucleic acid molecules according to the invention which code for an R3 protein are linked to a signal peptide in such a way that transcription and translation of said recombinant nucleic acid molecules give rise to a protein which has a plastidic signal peptide.

The present invention therefore furthermore relates to genetically modified plant cells according to the invention or genetically modified plants according to the invention, where the foreign nucleic acid molecule introduced into the plant cell or plant takes the form of a recombinant nucleic acid molecule, wherein said foreign nucleic acid molecule is a recombinant nucleic acid molecule where an R3-protein-encoding nucleic acid sequence is fused to nucleic acid sequences which code for a plastidic signal peptide so that the recombinant nucleic acid molecule codes for an R3 protein which additionally to its coding amino acid sequence has a plastidic signal peptide.

In the context of the present invention, the term "recombinant nucleic acid molecule" is understood as meaning a nucleic acid molecule which, in addition to nucleic acid molecules according to the invention which code for an R3 protein, comprises additional sequences which are not naturally present in a combination in which they are present in recombinant nucleic acids according to the invention. In this context, the addition of sequences mentioned may be any sequences; preferably, they take the form of regulatory sequences (promoters, termination signals, enhancers), especially preferably the form of regulatory sequences which are active in plant tissue, especially preferably in the form of regulatory sequences which are active in plant tissue in which storage starch is synthesized. Further preferred additional sequences are those sequences which code for signal peptides (signal sequences) which, as the result of translational fusion with protein-encoding sequences, bring about the translocation of the proteins according to the invention into subcellular compartments (vacuoles, plastids, mitochondria, nucleus, endoplasmic reticulum, cell wall). Especially important in the context of the present invention are signal sequences which code for plastidic signal peptides. Methods for generating recombinant nucleic acid molecules according to the invention are known to the skilled worker and comprise recombinant or molecular-biological methods such as, for example, the linkage of nucleic acid molecules by ligation, genetic recombination or the *de-novo* synthesis of nucleic acid molecules (see, for example, Sambrook et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition (2002), ISBN: 0471250929).

In the context of the present invention, the term "plastidic signal peptide" is to be understood as meaning a peptide which brings about the translocation of a protein into the lumen of plastids when the signal peptide is part of the amino acid sequence of a protein.

Regarding the expression in plants of the nucleic acid molecules according to the invention, it is possible, in principle, that the protein synthesized can be localized in any compartment of the plant cell. To achieve localization in a particular compartment, the coding region must, if appropriate, be linked with DNA sequences which ensure localization in the compartment in question. Such sequences are known (see, for example, Braun, EMBO J. 11 (1992), 3219-3227; Sonnewald, Plant J. 1 (1991), 95-106; Rocha-Sosa, EMBO J. 8 (1989), 23-29, Neuhaus and Rodgers, 1998, Plant Molecular Biology 38: 127-144). Here, the signal sequences are linked with the coding region of the protein in such a manner that the fused nucleic acid sequences of the signal peptide and those coding for the protein form, upon transcription, a shared open reading frame (translational fusion). Thus, after the translation of the RNA formed by the transcription of the fused nucleic acid sequences, a protein is formed which, in addition to the coding region of the protein, has a signal peptide.
In principle, any signal sequence which ensures the import of nuclear-encoded proteins into plastids may be used as plastidic signal sequence. Obtaining plastidic signal sequences is known to the skilled worker. Thus, for example, it is possible to use computer programs (1999, Emanuelsson et al., Protein Science 8, 978-984) to analyze coding protein sequences for the presence of a plastidic signal sequence. Computer programs which perform such analyses are both commercially available and available to the public on the internet (for example hftp://www.cbs.dtu.dklservices/ChloroP/).
For example, it is possible to use the plastidic signal sequence of the spinach ferredoxin:NADP+ oxidoreductase (FNR). This signal comprises the 5' untranslated region and the flanking transit peptide sequence of the cDNA of the plastidic protein ferredoxin:NADP+ oxidoreductase from spinach (nucleotide -171 to +165; Jansen et al., Current Genetics 13 (1988), 517-522).
Furthermore, it is possible to use for example the transit peptide of the waxy protein from maize plus the first 34 amino acids of the mature waxy protein (Klösgen et al., Mol Gen Genet. 217 (1989), 155-161) as plastidic signal sequence. Moreover, it is also possible to use the transit peptide of the waxy protein from maize (see above) without the first 34 amino acids of the mature waxy protein.
The use of the following plastidic signal sequences is also feasible: signal sequence of the ribulose bisphosphate carboxylase small subunit (Wolter et al., Proc. Natl. Acad. Sci. USA 85 (1988), 846-850; Nawrath et al., Proc. Natl. Acad. Sci. USA 91 (1994), 12760-12764); signal sequence of NADP-malate dehydrogenase (Gallardo et al., Planta 197 (1995), 324-332); signal sequence of glutathione reductase (Creissen et al., Plant J. 8 (1995), 167-175), signal sequence of EPSPS (US 5,188,642). It is preferred to use the signal sequence of the rice *dul* gene.
The recombinant nucleic acid molecule may comprise one or more, preferably two, signal sequences. The signal sequences can be present directly one after the other in the direction of transcription or else they can be separated from one another in each case by what is known as a spacer. Plastidic signal sequences which comprise two signal sequences are described for example in EP 0508909 and EP 0924299, US 5,510,471).

Surprisingly, it has been found that plant cells according to the invention and plants according to the invention synthesize a starch which is modified in comparison with the starch of corresponding non-genetically-modified wild-type plant cells or wild-type plants.

The plant cells according to the invention and plants according to the invention synthesize a modified starch whose physico-chemical properties, in particular whose starch phosphate content, is modified in comparison with starch synthesized in wild-type plant cells or wild-type plants so that it is better suited to specific applications.

The present invention therefore also comprises plant cells according to the invention and plants according to the invention which synthesize a starch which is modified in comparison with corresponding non-genetically-modified wild-type plant cells and wild-type plants, respectively.

In the context of the present invention, the term "modified starch" means that the starch has different physico-chemical properties in comparison with non-modified starch obtainable from corresponding wild-type plant cells or wild-type plants.

In a further embodiment of the present invention, plant cells according to the invention or plants according to the invention synthesize a starch whose starch phosphate content is increased in comparison with starch isolated from corresponding wild-type plant cells and wild-type plants, respectively.

A variety of methods have been described for determining the amount of starch phosphate. It is preferred to use the method described by Ritte et al. (2000, Starch/Starke 52, 179-185) for determining the amount of starch phosphate. The amount of starch phosphate is especially preferably carried out by means of ³¹P-NMR using the method described by Kasemusuwan and Jane (1996, Cereal Chemistry 73, 702-707).

The invention furthermore relates to genetically modified plants comprising plant cells according to the invention. Such plants can be generated from plant cells according to the invention by regeneration.

In principle, the plants according to the invention may take the form of plants of any plant species, i.e. both monocotyledonous and dicotyledonous plants. They preferably take the form of useful plants, i.e. plants which are grown by man for the purposes of nutrition or for technical, in particular industrial, purposes.

In a further embodiment, the plant according to the invention is a starch-storing plant.

In the context of the present invention, the term "starch-storing plants" means all plants with plant parts which comprise a storage starch such as, for example, maize, rice, wheat, rye, oats, barley, cassava, potato, sago, mung bean, pea or sorghum.

In the context of the present invention, the term "potato plant" or "potato" means plant species of the genome *Solanum,* especially tuber-producing species of the genus *Solanum* and in particular *Solanum tuberosum.*

In the context of the present invention, the term "wheat plant" means plant species of the genome *Triticum* or plants which have originated from hybridizations with plants of the genus *Triticum,* especially plant species of the genus *Triticum* which are grown in agriculture for commercial purposes or plants which have originated from hybridizations with plants of the genus *Triticum,* especially preferably *Triticum aestivum.*

In the context of the present invention, the term "maize plant" means plant species of the genus Zea, especially plant species of the genus Zea grown in agriculture for commercial purposes, especially preferably *Zea mais.*

In a further embodiment, the present invention relates to starch-storing plants according to the invention, preferably potato plants, especially preferably plants of the (systematic) family Poaceae. These plants especially preferably take the form of maize or wheat plants.

The present invention also relates to propagation material of plants according to the invention comprising plant cell according to the invention.

In this context, the term "propagation material" comprises those parts of the plant which are suitable for generating progeny via the vegetative or generative route. Those which are suitable for vegetative propagation are cuttings, callus cultures, rhizomes or tubers. Other propagation material comprises for example fruits, seeds, seedlings, protoplasts; cell cultures and the like. The propagation material preferably takes the form of tubers and especially preferably endosperm-comprising grains.

In a further embodiment, the present invention relates to harvestable plant parts of plants according to the invention, such as fruits, storage and other roots, flowers, buds, shoots or stalks, preferably seeds, grains or tubers, these harvestable parts comprising plant cells according to the invention.

The present invention furthermore also describes a method for generating genetically modified plants according to the invention wherein
a) a plant cell is genetically modified, the genetic modification leading to an increase in the activity of an R3 protein in comparison with corresponding non-genetically-modified wild-type plant cells;
b) a plant is regenerated from plant cells of step a);
c) and, if appropriate, further plants are generated with the aid of the plants of step b).

The present invention relate to a method for generating a genetically modified plant, wherein
a) a plant cell is transfromed, the transformation leading to an increase in the activity of at least one protein coding a protein which transfers a phosphate residue from a nucleoside triphosphate to starch in comparison with corresponding non-genetically-modified wild-type plant cells and consists in the introduction of a foreign nucleic acid molecule coding a protein which transfers a phosphate residue from a nucleoside triphosphate to starch selected from the group consisting of I
   i) nucleic acid molecules which code for a protein with the amino acid sequence shown in SEQ ID NO 3;
   ii) nucleic acid molecules which code for a protein which comprises the amino acid sequence which is encoded by the insertion in DSM 16587 or by the insertion in DSM 1664;
   iii) nucleic acid molecules which code for a protein whose amino acid sequence has at least 60% identity with the amino acid sequence shown In SEQ ID NO 3;
   iv) nucleic acid molecules which code for a protein whose sequence has at least 60% identity with the amino acid sequence which is encoded by the insertion in plasmid DSM 16587 or by the insertion in plasmid DSM 1664;
   v) nucleic acid molecules which comprise the nucleotide sequence shown in SEQ ID NO 1 or SEQ ID NO 2 or a complementary sequence;
   vi) nucleic acid molecules which comprise the nucleotide sequence of the insertion present in plasmid DSM 16587 or in plasmid DSM 1664;
   vii) nucleic acid molecules which have at least 70% identity with the nucleic acid sequences described in i), ii), v) or vi);
   viii) nucleic acid molecules which hybridize under stringent conditions with at least one strand of the nucleic acid molecules described in i), ii), v) or vi);
   iix) nucleic acid molecules whose nucleotide sequence, as the result of the degeneracy of the genetic code, deviates from the sequence of the nucleic acid molecules mentioned in i), ii), v) or vi); and
   ix) nucleic acid molecules which constitute fragments, allelic variants and/or derivatives of the nucleic acid molecules mentioned In i), ii), iii), iv), v), vi), vii), viii) or iix),
   into the genome of the plant cell, wherein said foreign nucleic acid molecule is a recombinant nucleic acid molecule comprising additional sequences which are not naturally present in a combination in which they are present in recombinant nucleic acids in which a nucleic acid sequence encoding a protein which transfers a phosphate residue from a nucleoside triphosphate to starch is fused with nucleic acid sequence which code for a plastidic signal peptide so that the recombinant nucleic acid molecule codes for a protein which transfers a phosphate residue from a nucleoside triphosphate to starch in addition to its coding amino acid sequence has a plastidic signal peptide,
b) a plant is regenerated from plant cells of step a); and
c) if appropriate, further plants are generated with the aid of the plants of step b).

As regards the genetic modification introduced into the plant cell in accordance with step a), this may in principle take the form of any type of modification which leads to an increase in the activity of an R3 protein.
The plants can be regenerated in accordance with step b) by methods known to the skilled worker (for example described in "Plant Cell Culture Protocols", 1999, edt. by R.D. Hall, Humana Press, ISBN 0-89603-549-2).

The generation of further plants in accordance with step c) of the method according to the invention can be performed for example by vegetative propagation (for example via cuttings, tubers or via callus culture and regeneration of intact plants) or via generative propagation. In this context, generative propagation generally takes place under controlled conditions, i.e. selected plants with specific characteristics are hybridized with one another and multiplied. Selection preferably takes place in such a manner that the further plants which are obtained in accordance with step c) comprise the genetic modification which has been introduced in step a).

In the method according to the invention for the generation of a genetically modified plant, the genetic modification of a plant cell in step a) leads to an increase in the activity of an R3 protein in plastids of said plant cell in comparison with plastids of corresponding non-genetically-modfied wild-type plant cells.

In the method according to the invention, the genetic modification consists in introducing, into the genome of the plant cell, a foreign nucleic acid molecule according to the invention or a recombinant nucleic acid molecule according to the invention, the presence or expression of said foreign nucleic acid molecule or said recombinant nucleic acid molecule leading to an increased activity of an R3 protein in the cell.

In the method according to the invention, the genetic modification consists in the introduction of a foreign nucleic acid molecule into the genome of the plant cell, the foreign nucleic acid molecule coding for an R3 protein.

In the method according to the invention, the genetic modification consists in the introduction of a foreign nucleic acid molecule into the genome of the plant cell, said foreign nucleic acid molecule being a recombinant nucleic acid molecule where an R3-protein-encoding nucleic acid sequence is fused with a second nucleic acid sequence which codes for a plastidic signal peptide so that the recombinant nucleic acid molecule codes for an R3 protein which, in addition to its coding amino acid sequence, has a plastidic signal peptide.

In a further embodiment, the method according to the invention for the generation of a genetically modified plant according to the invention is used for generating starch-storing plants.

In a further embodiment, the method according to the invention is used for generating potato, maize or wheat plants according to the invention.

In the method according to the invention, the foreign nucleic acid molecule is selected from the group consisting of
a) nucleic acid molecules which code for a protein with the amino acid sequence shown in SEQ ID NO. 3;
b) nucleic acid molecules which code for a protein which comprises the amino acid sequence which is encoded by the insertion in plasmid DSM 16587 or by the insertion in plasmid DSM 16645;
c) nucleic acid molecules which code for a protein whose amino acid sequence has at least 60% identity with the amino acid sequence shown in SEQ ID NO 3;
d) nucleic acid molecules which code for a protein whose sequence has at least 60% identity with the amino acid sequence which is encoded by the insertion in plasmid DSM 16587 or by the insertion in plasmid DSM 16645;
e) nucleic acid molecules which comprise the nucleotide sequence shown in SEQ ID NO 1 or SEQ ID NO 2 or a complementary sequence;
f) nucleic acid molecules which comprise the nucleoside sequence of the insertion present in plasmid DSM 16587, or in plasmid DSM 16645;
g) nucleic acid molecules which have at least 70% identity with the nucleic acid sequences described in a), b), e) or f);
h) nucleic acid molecules which hybridize under stringent conditions with at least one strand of the nucleic acid molecules described in a), b), d), e) or f);
i) nucleic acid molecules whose nucleotide sequence, as the result of the degeneracy of the genetic code, deviates from the sequence of the nucleic acid molecules mentioned in a), b), e) or f); and
j) nucleic acid molecules which constitute fragments, allelic variants and/or derivatives of the nucleic acid molecules mentioned in a), b), c), d), e), f), g), h) or i).

In the method according to the invention, the nucleic acid molecule according to the invention is a recombinant nucleic acid molecule, where said foreign nucleic acid molecule is a recombinant nucleic acid molecule in which an R3-protein-encoding nucleic acid sequence is fused with nucleic acid sequences which code for a plastidic signal peptide so that the recombinant nucleic acid molecule codes for an R3 protein which in addition to its coding amino acid sequence has a plastidic signal peptide.

In a further embodiment, the present invention relates to a method according to the invention where the genetically modified plant synthesizes a starch which is modified in comparison with starch from non-genetically-modified wild-type plants.

In a further embodiment of the method according to the invention, the plants according to the invention synthesize a modified starch with an elevated starch phosphate content in comparison with starch isolated from corresponding wild-type plants.

The present invention also relates to the plants obtainable by methods according to the invention.

Surprisingly, it has been found that starch isolated from plant cells according to the invention or plants according to the invention with an increased activity of an R3 protein in the plastids present in them synthesize a modified starch.

In particular the increased amounts of starch phosphate of starches according to the invention confer surprising and advantageous properties to the starches. As the result of the increased amount of starch phosphate, starches according to the invention bear an increased amount of charged groups which have an essential effect on the functional properties of the starch. Starch which bears charged functional groups can be employed in particular in the paper industry where it is used for the coating of paper. Paper which is coated with charged molecules which additionally have good adhesive properties is particularly suitable for absorbing colors such as, for example, ink, printing inks and the like.

Also described are modified starches obtainable from plant cells according to the invention or plants according to the invention, from propagation material according to the invention or from harvestable plant parts according to the invention.

Further described is modified starch from starch-storing plants, preferably from potato plants, especially preferably from starch-storing plants of the (systematic) family Poaceae, particularly preferably from maize or wheat plants.

The present invention further relates to a method for producing a modified starch comprising the step of extracting the starch from a plant cell according to the invention or a plant according to the invention, from propagation material according to the invention of such a plant and/or from harvestable plant parts according to the invention of such a plant, preferably from starch-storing parts according to the invention of such a plant. Preferably, such a method also comprises the step of harvesting the cultured plants or plant parts and/or of the propagation material of these plants prior to extraction of the starch, and especially preferably furthermore the step of culturing plants according to the invention prior to harvesting.

Methods for extracting the starch from plants or from starch-storing parts of plants are known to the skilled worker. Furthermore, there are described methods for extracting the starch from various starch-storing plants, for example in Starch: Chemistry and Technology (Ed.: Whistler, BeMiller and Paschall (1994), 2nd Edition, Academic Press Inc. London Ltd; ISBN 0-12-746270-8; see, for example, Chapter XII, page 412-468: maize and sorghum starches: production; by Watson, Chapter XIII, pages 469-479: tapioca, arrowroot and sago starches: production; by Corbishley and Miller, Chapter XIV, pages 479-490: potato starch: production and uses; by Mitch; Chapter XV, pages 491 to 506: wheat starch: production, modification and uses; by Knight and Oson; and Chapter XVI, pages 507 to 528: rice starch: production and uses; by Rohmer and Klem; maize starch: Eckhoff et al., Cereal Chem. 73 (1996), 54-57, the extraction of maize starch on an industrial scale is generally achieved by what is known as wet milling). Devices normally used in processes for extracting starch from plant material are separators, decanters, hydrocyclones, spray dryers and fluidized-bed dryers.

In the context of the present invention, the term "starch-storing plants" is understood as meaning those parts of a plant in which starch is stored as a depot to survive for long periods, in contrast to transitory leaf starch. Preferred starch-storing plant parts are, for example, tubers, storage roots and grains, especially preferably being grains comprising endosperm, especially preferably being grains comprising an endosperm of maize or wheat plants.

In the context of the present invention, the term "native starch" means that the starch is isolated from plants according to the invention, harvestable plant parts according to the invention, starch-storing parts according to the invention or plant propagation material according to the invention by methods known to the skilled worker.

Another subject matter of the present invention is the use of plant cells according to the invention or plants according to the invention for the production of a modified starch.

The skilled worker knows that the properties of starch can be modified by, for example, thermal, chemical, enzymatic or mechanical derivatization. Derivatized starches are particularly suitable for a variety of applications in the food and/or nonfood sectors. The starches are better suited as starting material for the production of derivatized starches than conventional starches since, as the result of their higher starch phosphate content, they have a higher content of reactive functional groups.

The term "derivatized starch" is understood as meaning a modified starch whose characteristics are modified after its isolation from plant cells by means of chemical, enzymatic, thermal or mechanical methods.

The derivatized starches can be starch ethers, in particular starch alkyl ethers, O-allyl ethers, hydroxyalkyl ethers, O-carboxymethyl ethers, nitrogen-containing starch ethers, phosphate-containing starch ethers or sulfur containing starch ethers.

The derivatized starches can be crosslinked starches.

The derivatized starches can be starch graft polymers.

The derivatized starches can be oxidized starches.

Further the derivatized starches can be starch esters, in particular starch esters which have been introduced into the starch using organic acids. They are preferably phosphate, nitrate, sulfate, xanthate, acetate or citrate starches.

Derivatized starches are suitable for various applications in the pharmacological industry, in the food and/or nonfood sectors. Methods for the production of derivatized starches are known to the skilled worker and described sufficiently in the general literature. An overview over the production of derivatized starches is found for example in "Com, Chemistry and Technology", (1987, eds. Watson and Ramstad, Chapter 16, 479-499).

Starch-storing parts of plants are frequently processed into flours. Examples of parts of plants from which flours are produced are, for example, tubers of potato plants and grains of cereal plants. To produce flours from cereal plants, the endosperm-containing grains of these plants are ground and sieved. Starch is a main constituent of the endosperm. In the case of other plants which do not comprise an endosperm, but other starch-storing parts such as, for example, tubers or roots, flour is frequently produced by comminuting, drying and subsequently grinding the storage organs in question. The starch of the endosperm or present in starch-storing parts of plants is an important constituent of the flour which is produced from the plant parts in question. This is why the properties of flours are affected by the starch present in the flour in question. Plant cells according to the invention and plants according to the invention synthesize a starch which is modified in comparison with corresponding non-genetically-modified wild-type plant cells or non-genetically-modified wild type plants. Flours produced from plant cells according to the invention, plants according to the invention, propagation material according to the invention or harvestable parts according to the invention have therefore modified properties. The properties of flours can also be influenced by mixing starch with flours or by mixing flours which have different properties.

Also described are flours which are produced from plant cells according to the invention, plants according to the invention, from starch-storing parts of plants according to the invention, from propagation material according to the invention or from harvestable plant parts according to the invention. Preferred starch-storing parts of plants according to the invention are tubers, storage roots and endosperm-containing grains. Preferably, tubers are tubers of potato plants and grains are grains of plants of the (systematic) family Poaceae; especially preferably, grains are grains of maize or wheat plants.

In the context of the present invention, the terms "flour" is understood as meaning a powder obtained by grinding plant parts. If appropriate, plant parts are dried before grinding and comminuted and/or sieved after grinding.

As the result of the starch present in them, which has a modified phosphate content, flours according to the invention are distinguished in particular by their increased water-binding capacity. This is desired for example for a number of applications when processing flours in the food industry, in particular in the production of baked goods.

The present invention furthermore relates to a method for the production of flours, comprising the step of grinding plant cells according to the invention, plants according to the invention, parts of plants according to the invention, starch-storing parts of plants according to the invention, propagation material according to the invention or harvestable material according to the invention.

Flours can be produced by grinding starch-storing parts of plants according to the invention. The skilled worker knows how to produce flours. Preferably, a method for the production of flours also comprises the step of harvesting the cultured plants or plant parts and/or the propagation material or the starch-storing parts of these plants prior to grinding, and especially preferably furthermore the step of culturing plant according to the invention prior to harvesting.

In the context of the present invention, the term "parts of plants" are to be understood to be all parts of the plant which, in the totality of their constituents, form a complete plant. Examples of parts of plants are shoots, leaves, rhizomes, roots, beet, tubers, pods, seeds or grains.

In a further embodiment of the present invention, the method for the production of flours comprises the processing of plants according to the invention, of starch-storing parts of plants according to the invention, of propagation material according to the invention or of harvestable material according to the invention prior to grinding.
In this context, the processing may be for example a thermal treatment and/or drying. Thermal treatment followed by drying of the material which has been subjected to the thermal treatment is applied for example in the production of flours from storage roots or tubers such as, for example, potato tubers prior to grinding. Plants according to the invention, starch-storing parts of plants according to the invention, propagation material according to the invention or harvestable material according to the invention prior to grinding can likewise constitute the processing for the purposes of the present invention. The removal of plant tissue such as, for example, of husks of the grains, prior to grinding, is also a processing prior to grinding for the purposes of the present invention.

In a further embodiment of the present invention, the method for the production of flours after grinding comprises a processing of the ground material.
For example, the ground material can be sieved after grinding, for example to produce different types of flours.

The present invention furthermore relates to the use of genetically modified plant cells according to the invention or plants according to the invention for the production of flours.

Another object of the present invention is to provide means such as, for example, DNA molecules, for generating plant cells according to the invention and plants according to the invention which, in comparison to non-genetically-modified wild-type plant cells or wild-type plants, synthesize a modified starch.

Thus, the present invention also relates to a nucleic acid molecule coding for a protein with the enzymatic activity of a protein which transfers a phosphate residue from a nucleoside triphosphate to starch, selected from the group consisting of
a) a nucleic acid molecule which codes for a protein with the amino acid sequence shown in SEQ ID NO 3;
b) a nucleic acid molecule which codes for a protein whose amino acid sequence has at least 60% identity with the amino acid sequence shown in SEQ ID NO 3;
c) a nucleic acid molecule which comprises the nucleotide sequence shown In SEQ ID No. 1 or SEQ ID NO 2 or a sequence which is complementary to these sequences;
d) a nucleic acid molecule which has at least 70% identity with the nucleic acid sequences described in a) or c);
e) a nucleic acid molecule which hybridizes under stringent conditions with at least one strand of the nucleic acid molecule described in a) or c);
f) a nucleic acid molecule whose nucleotide sequence, as the result of the degeneracy of the genetic code, deviates from the sequence of the nucleic acid molecules mentioned in a) or c); and
g) a nucleic acid molecule which constitutes a fragment, allelic variant and/or derivative of the nucleic acid molecules mentioned in a), b), c), d), e) or f),
and wherein said nucleic acid molecule is a recombinant nucleic acid molecule comprising additional sequences which are not naturally present In a combination in which they are present in recombinant nucleic acids encoding a protein which transfers a phosphate residue from a nucleoside triphosphate to starch is fused to nucleic acid sequences which code for a plastidic signal peptide.

The nucleic acid molecules according to the invention may, in principle, be derived from any plant, preferably from *Arabidopsis* plants.

The present invention furthermore describes nucleic acid molecules of at least 21, preferably more than 50 and especially preferably more than 200 nucleotides in length, which nucleic acid molecules hybridize specifically with at least one nucleic acid molecule according to the invention. In the present context, to hybridize specifically means that these molecules hybridize with nucleic acid molecules which code for a protein according to the invention, but not with nucleic acid molecules which code for other proteins. The invention in particular relates to those nucleic acid molecules which hybridize with transcripts of nucleic acid molecules according to the invention and can therefore prevent the translation of the latter. Such nucleic acid molecules which hybridize specifically with the nucleic acid molecules according to the invention can be, for example, components of antisense constructs, RNAI constructs, cosuppression constructs or ribozymes, or can be used as primers for amplification by means of PCR.

The invention describes recombinant nucleic acid molecules comprising a nucleic acid molecule according to the invention.

The present invention describes a recombinant nucleic acid molecule in which an R3-protein-encoding nucleic acid sequence is fused to nucleic acid sequences which code for a plastidic signal peptide so that the recombinant nucleic acid molecule codes for an R3 protein which, in addition to its coding amino acid sequence, comprises a plastidic signal peptide.

A further embodiment of recombinant nucleic acid molecules according to the invention, of the present invention, are vectors, in particular plasmids, cosmids, viruses, bacteriophages and other vectors conventionally used in genetic engineering which comprise the above-described nucleic acid molecules according to the invention.

In a further embodiment, the nucleic acid molecules according to the invention which are present in the vectors are linked with regulatory sequences which initiate the expression in prokaryotic or eukaryotic cells. In this context, the term "expression" can mean transcription or else transcription and translation. The nucleic acid molecules according to the invention can be present in sense orientation and/or in antisense orientation relative to the regulatory sequences.

Regulatory sequences for expression in prokaryotic organisms, for example *E. coli,* and in eukaryotic organisms are described extensively in the literature, in particular those for expression in yeast, such as, for example, *Saccharomyces cerevisiae.* An overview over various systems for the expression of proteins in different host organisms can be found for example in Methods in Enzymology 153 (1987), 383-516 and in Bitter et al. (Methods in Enzymology 153 (1987), 516-544).

The present invention furthermore relates to a host cell, in particular to a prokaryotic or eukaryotic cell, which is genetically modified with a nucleic acid molecule according to the invention and/or with a vector according to the invention, and to cells which are derived from such host cells and which comprise the genetic modification according to the invention.

The host cells may be bacterial cells (for example *E*. *coli,* bacteria of the genus *Agrobacterium,* in particular *Agrobacterium tumefaciens* or *Agrobacterium rhizogenes*) or fungal cells (for example yeast, in particular *S*. *cerevisiae, Agaricus,* in particular *Agaricus bisporus, Aspergillus, Trichoderma*), or plant or animal cells. In the present context, the term "transformed" means that the cells according to the invention are genetically modified with a nucleic acid molecule according to the invention in as far as they comprise at least one nucleic acid molecule according to the invention in addition to their natural genome. This nucleic acid molecule according to the invention may be present in the cell in free form, if appropriate as a self-replicating molecule, or else it may be stably integrated into the genome of the host cell.
The host cells are preferably microorganisms. For the purposes of the present application, this is understood as meaning all bacteria and all protists (for example fungi, in particular yeasts and algae) as they are defined for example in Schlegel "Allgemeine Mikrobiologie" [General Microbiology] (Georg Thieme Verlag (1985), 1-2).

The host cells according to the invention are preferably plant cells. In principle, they may take the form of plant cells from any desired plant species, i.e. both monocotyledonous and dicotyledonous plants. They preferably take the form of plant cells from agricultural crop plants, i.e. from plants which are cultured by man for the purposes of nutrition or for technical, in particular industrial, purposes. The invention preferably relates to plant cells and plants from starch-storing plants (maize, rice, wheat, rye, oats, barley, cassava, potato, sago, mung bean, pea or sorghum), preferably plant cells from potato plants, especially preferably plant cells from plants of the (systematic) family Poacea, with plant cells from maize or wheat plants being especially preferred.
The present invention also relates to compositions comprising a nucleic acid molecule according to the invention, a recombinant nucleic acid molecule according to the invention or a vector according to the invention. Preferred are compositions according to the invention comprising a nucleic acid molecule according to the invention, a recombinant nucleic acid molecule according to the invention or a vector according to the invention and a host cell. The host cell especially preferably takes the form of a plant cell, especially preferably the form of a cell of a maize or wheat plant. In these compositions, the recombinant nucleic acid molecule according to the invention exists outside the host cell, i.e. it is located outside the inner part of the host cell which is surrounded by a cytoplasm membrane.

A further aspect of compositions according to the invention relates to compositions which can be used for the generation of host cells according to the invention, preferably for the generation of plant cells according to the invention. This preferably takes the form of a composition comprising a nucleic acid molecule according to the invention, a recombinant nucleic acid molecule according to the invention or a vector according to the invention and a biolistic support which is suitable for introducing a nucleic acid molecule according to the invention into a host cell. Preferred biolistic supports are particles made of tungsten, gold or plastics.

A further embodiment of compositions according to the invention relates to compositions comprising a nucleic acid molecule according to the invention, a recombinant nucleic acid molecule according to the invention or a vector according to the invention and a plant cell and a synthetic culture medium. In addition to nucleic acid molecules according to the invention, plant cells and synthetic culture medium, such compositions preferably also comprise polyethylene glycol (PEG). In these compositions, the recombinant nucleic acid molecule according to the invention is present outside the plant cell, i.e. it is located outside the internal part of the plant cell which is surrounded by a cytoplasm membrane.
Synthetic culture media which are suitable for culturing and/or for the transformation of plant cells are known to the skilled worker and are for example described extensively in the literature. Many different synthetic culture media can also be purchased commercially in the specialist trade (for example DUCHEFA Biochemie B.V. Belgium).

A further embodiment of compositions according to the invention relates to compositions which are used to identify nucleic acids according to the invention. In addition to a nucleic acid molecule according to the invention, recombinant nucleic acid molecule according to the invention or vector according to the invention, such compositions preferably comprise further nucleic acid molecules, in particular nucleic acid molecules of vegetable origin, which can be present in the form of genomic DNA, mRNA or as clones in what are known as DNA libraries. Preferred are DNA libraries which exist as cosmids, phagemids, plasmids, YACs or BACs. The DNA libraries may comprise both genomic DNA or cDNA. The nucleic acid molecules according to the invention, recombinant nucleic acid molecules according to the invention or vectors according to the invention are preferably, employed as hybridization probes in these compositions.

A further subject matter of the present invention are proteins selected from the group consisting of
a) proteins which comprise the amino acid sequence shown in SEQ ID NO 3;
b) proteins which are encoded by the coding region of the DNA inserted in the plasmid pIR138-210 or in the plasmid pIR139-210; or
c) proteins which have at least 60% identity with the amino acid sequence of the proteins mentioned in a) or b).

The present invention furthermore relates to a protein according to the invention which has a molecular weight of from 120 kDa to 145 kDa, preferably from 120 kDa to 140 kDa, especially preferably from 125 kDa to 140 kDa, especially preferably from 130 kDa to 135 kDa, derived from the amino acid sequence.

A further embodiment of the present invention relates to a protein according to the invention which has a phosphohistidine domain.

In a further embodiment, the present invention relates to proteins with starch-phosphorylating activity, where the encoded protein has at least 70%, preferably at least 80%, particularly preferably at least 90% and especially preferably at least 95% identity with the amino acid sequence shown in SEQ ID NO 3 or with the amino acid sequence, of an R3 protein, encoded by the insertion in plasmid pIR138-210 or by the insertion in plasmid pIR139-210.

A further embodiment of the present invention relates to a protein according to the invention wherein the amino acid sequence which codes for the protein has a phosphohistidine domain. Preferably, the protein according to the invention has a phosphohistidine domain which has at least 85%, especially at least 88%, preferably at least 91% and particularly preferably at least 94% and especially preferably at least 97% identity with the amino acid sequence shown in SEQ ID NO 4.

A preferred embodiment of the process according to the invention relates to recombinant proteins which comprise an R3-protein-encoding amino acid sequence which is fused to amino acid sequences which code for a plastidic signal peptide.

In the context of the present invention, the term "recombinant protein" is to be understood as meaning a protein which, besides amino acid sequences coding for an R3 protein according to the invention, comprises additional amino acid sequences, where the combination of R3-protein-encoding amino acid sequences and of said additional amino acid sequences does not naturally exist in the combination in which it exists in recombinant proteins according to the invention. In this context, the abovementioned additional amino acid sequences may be any amino acid sequences. Preferably, they take the form of amino acid sequences which code for signal peptides which, as the result of translational fusion with R3-protein-encoding amino acid sequences, bring about a translocation of the recombinant proteins in question into subcellular compartments (vacuoles, plastids, microchondria, nucleus, endoplasmic reticulum, cell wall). Especially important in the context of the present invention are amino acid sequences which code for plastidic signal peptides. Methods for the generation of recombinant proteins according to the invention, are known to the skilled worker and comprise recombinant methods such as, for example, the transcription and translation of recombinant nucleic acid molecules which code for recombinant proteins.

Sources of amino acid sequences which code for plastidic signal peptides have already been mentioned above in the context of the term "recombinant nucleic acid molecule".

In a further embodiment, the present invention relates to a protein according to the invention, the protein being derived from an *Arabidopsis* plant.

In a further embodiment, the invention also relates to proteins which are encoded by nucleic acid molecules according to the invention.

### Description of the sequences

SEQ ID NO 1: Nucleic acid sequence comprising the coding region of the *Arabidopsis thaliana* R3 protein. This sequence is inserted in the plasmid pIR139-210.
SEQ ID NO 2: Nucleic acid sequence comprising the coding region of the *Arabidopsis thaliana* R3 protein in translational fusion with the signal sequence coding for the plastidic signal peptide of the *dul I* gene from *Oryza sativa.* This sequence is inserted in the plasmid pIR138-210.
SEQ ID NO 3: Amino acid sequence coding for the fusion protein consisting of the amino acid sequence coding for the *Arabidopsis thaliana* R3 protein and the amino acid sequence coding for the plastidic signal peptide of the *dul* / gene (TrEMBL Acc. No.: Q621 D6) from *Oryza sativa.* This sequence can be derived from the nucleic acid sequence shown in SEQ ID NO 2.
SEQ ID NO 4: Amino acid sequence of the phosphohistidine domain which is present in the amino acid sequence coding for the *Arabidopsis thaliana* R3 protein.

### General methods

The following text will describe methods which can be used for implementing the methods according to the invention. These methods are specific embodiments of the present invention, but do not limit the present invention to these methods. The skilled worker knows that the invention can be implemented in the same manner by modifying the methods described and/or by replacing individual parts of the methods by alternative parts of the methods.

### 1. Recombinant expression of an identified starch-phosphorylating protein

### a) Generation of a bacterial expression vector comprising a cDNA which codes for a starch-phosphorylating protein

The cDNA coding for a starch-phosphorylating protein can be amplified by means of polymerase chain reaction (PCR) for example using mRNA or poly-A-plus-mRNA from plant tissues as template. To this end, a reverse transcriptase is initially used for generating a cDNA strand which is complementary to the mRNA coding for a starch-phosphorylating protein before the cDNA strand in question is amplified by means of DNA polymerase. Kits comprising substances, enzymes and instructions for carrying out PCR reactions are commercially available (for example SuperScript^{™} One-Step RT-PCR System, Invitrogen, Prod. No.: 10928-034). The cDNA which has been amplified and which codes for a starch-phosphorylating protein can subsequently be cloned into a bacterial expression vector, for example pest^{™}17 (Invitrogen). pDEST^{™}17 comprises the T7 promoter which is used for initiating the transcription of the T7 RNA polymerase. Furthermore, the expression vector pDEST^{™}17 comprises, in the 5' direction from the T7 promoter, a Shine-Dalgamo sequence followed by a start codon (ATG) and by what is known as a His tag. This His tag consists of six consecutive codons, each of which codes for the amino acid histidine, and is located in the reading frame of the abovementioned start codon. A cDNA which codes for a starch-phosphorylating protein is cloned into pDEST^{™}17 in such a way that a translational fusion is generated between the codons for the start codon, the His tag and the cDNA coding for a starch-phosphorylating protein. After transcription, initiated at the T7 promoter, and subsequent translation, a starch-phosphorylating protein is obtained which comprises, at its N terminus, additional amino acids including the His tag.
However, other vectors which are suitable for expression in microorganisms can also be used for expressing a starch-phosphorylating protein. Expression vectors and matching expression strains are known to the skilled worker, and suitable combinations are also commercially available.

### b) Generation of expression clones in Escherichia coli

First, a suitable transformation-competent *E. coli* strain which chromosomally codes for a T7 RNA polymerase is transformed with the expression plasmid generated as described in step a) and the strain is subsequently incubated overnight at 30°C on agar-solidified nutrient medium. Suitable expression strains are, for example, BL21 strains (Invitrogen Prod. No.: C6010-03 which chromosomally code for a T7 RNA polymerase under the control of an IPTG-inducible promoter (lacZ).
Bacterial colonies which are the result of the transformation step can be analyzed by methods known to the skilled worker for the presence of the desired expression plasmid comprising a cDNA which codes for the starch-phosphorylating protein. This gives expression clones.

### c) Expression of a starch-phosphorylating protein in Escherichia coli

First, a preculture is prepared. To this end, an expression clone obtained in step b) is inoculated into 30 ml of Terrific Broth (TB medium) comprising an antibiotic for selection for the presence of the expression plasmid, and the culture is incubated overnight at 30°C with shaking (250 rpm).
Then, a main culture for expressing a starch-phosphorylating protein is prepared. To this end, 1-liter. Erlenmeyer flasks, each of which comprises 300 ml of TB medium which has been prewarmed to 30°C and an antibiotic for the selection for the presence of the expression plasmid are inoculated in each case with 10 ml of a suitable preculture and incubated at 30°C with shaking (250 rpm) to an optical density of approx. 0.8 (measured at a wavelength of 600 nm; OD₆₀₀).
If, to express a starch-phosphorylating protein, an expression plasmid was used in which the expression of the starch-phosphorylating protein is initiated by means of an inducible system (for example the expression vector pDEST^{™}17 in BL21 *E. coli* strains, inducible by IPTG), the inductor in question (for example IPTG) is added once an OD₆₀₀ of approx. 0.8 has been reached in the main culture. After addition of the inductor, the main culture is incubated at 30°C with shaking (250 rpm) until an OD₆₀₀ of approx. 1.8 has been reached. Thereafter, the main culture is ice-cooled for 30 minutes, and the cells of the main culture are then separated from the culture medium by centrifugation (10 minutes at 4000xg and 4°C).

### 2. Purification of a starch-phosphorylating protein

### a) Disruption of cells expressing a starch-phosphorylating protein

The cells obtained after centrifugation in step c), Item 1, General Methods, are resuspended in lysis buffer, where approximately 4 ml of lysis buffer are added to approximately 1 g of cells. Thereafter, the resuspended cells are incubated on ice for 30 minutes and then disrupted with the aid of a sonicator (Baudelin Sonoplus UW 2070, Baudelin electronic, Berlin, settings: Cycle 6,70%, 1 minute) with continuous ice-cooling. Care must be taken that the cell suspension is not unduly heated during the sonication. The suspension obtained after sonication is centrifuged (12 minutes at 20000 xg, 4°C), and the supernatant obtained after centrifugation is filtered through a filter with a pore size of 45 µm.

### b) Purification of the starch-phosphorylating protein,

If the starch-phosphorylating protein expressed in *E. coli* cells is a fusion protein with a His tag, it can be purified with the aid of nickel ions, to which the His tag binds with high affinity. To this end, 25 ml of the filtrate obtained in step d) is treated with 1 ml of Ni-agarose slurry (Qiagen, Prod. No.: 30210) and the mixture is incubated on ice for 1 hour. Thereafter the mixture of Ni-agarose slurry and filtrate is passed over a polysterene column (Pierce, Prod. No.: 29920). The run-through of the column is discarded. The column is first washed by applying 8 ml of lysis buffer, the run-through again being discarded. The starch-phosphorylating protein is then eluded by fractionally applying two portions of in each case 1 ml of E1 buffer, followed by one portion of 1 ml of E2 buffer and then five portions of in each case 1 ml of E3 buffer to the column. The run-through obtained when applying the individual fraction of the elution buffers in question (E1, E2 and E3 buffers) is collected in separate fractions. Aliquots of these fractions are subsequently analyzed by means of denaturing SDS-acrylamide gel electrophoresis, followed by staining with Coomassie Blue. The fractions which comprise the starch-phosphorylating protein in sufficient amount and satisfying purity are combined and concentrated at 4°C with the aid of pressure filtration. Pressure filtration can be effected for example with the aid of an Amicon cell (Amicon Ultrafiltration Cell, Model 8010, Prod. Nr.: 5121) using a Diaflo PM30. membrane (Millipore, Prod, No.: 13212) at 4°C. However, other methods with which the skilled worker is familiar may also be used for the concentration step.

### c) Composition of buffers used

| | | |
|---|---|---|
| Lysis buffer: | 50 mM | HEPES |
| | 300 mM | NaCl |
| | 10 mM | imidazole |
| pH 8.0 | (adjust with NaOH) | |
| 1 mg/ml | lysozyme (add immediately before using the buffer) | |
| ¼ tablet Product per 10 ml protease inhibitors Complete EDTA free, (Roche No.: 1873580) (add immediately before using the buffer) | | |

| | | |
|---|---|---|
| Elution buffer E1: | 50 mM | HEPES |
| | 300 mM | NaCl |
| | 50 mM | imidazole |
| | pH 8.0 (adjust with NaOH) | |

| | | |
|---|---|---|
| Elution buffer E2: | 50 mM | HEPES |
| | 300 mM | NaCl |
| | 75 mM | imidazole |
| | pH 8.0 (adjust with NaOH) | |

| | | |
|---|---|---|
| Elution buffer E3: | 50 mM | HEPES |
| | 300 mM | NaCl |
| | 250 mM | imidazole |
| | pH 8.0 (adjust with NaOH) | |

### 3. Method for detecting starch-phosphorylating activity of a protein

### a) Incubation of proteins with starch and/or nonphosphorylated starch.

To detect whether a protein has a starch-phosphorylating activity, test proteins can be incubated with starch and with radiolabeled nucleoside triphosphates. To this end, approx. 5 mg of starch together with the test protein (0.01 µg to 5.0 µg per mg of starch employed) are incubated with shaking at room temperature for 10 minutes to 30 minutes in 500 µl of phosphorylation buffer. Thereafter, the reaction is stopped by adding SDS to a concentration of 2% (weight/volume). The starch granules which are present in the reaction mixture are separated off by centrifuging (1 minute, 13000 xg) and washed once with 900 µl of 2% strength SDS solution and four times with in each case 900 µl of a 2 mM nucleoside triphosphate solution. Each wash step is carried out for 15 minutes at room temperature with shaking. After each wash step, the starch granules are separated from the wash buffer in question by centrifugation (1 minute, 13000xg).
When carrying out an experiment for detecting starch-phosphorylating activity of a protein, further reaction mixtures which comprise no protein, or inactivated protein, but which are otherwise treated in the same manner as the reaction mixtures described, should additionally be tested to act as what are known as controls.

### b) Determining the amount of phosphate residues incorporated into the starch as the result of enzymatic activity

The starch granules obtained in step a) can be tested for the presence of radiolabelled phosphate residues. To this end, the starch in question is resuspended in each case in 100 µl of water, treated with in each case 3 ml of scintillation cocktail (for example Ready Safe^{™}, BECKMANN Coulter) and subsequently analyzed with the aid of a scintillation counter (for example LS 6500 Multi-Purpose Scintillation counter, BECKMANN COULTER^{™}).

### d) Composition of buffers used

| | | |
|---|---|---|
| Phosphorylation buffer: | 50 mM | HEPES/KOH, pH 7.5 |
| | 1 mM | EDTA |
| | 6 mM | MgCl₂ |
| | 0.01 to 0.5 mM ATP | |
| | 0.2 to 2 µCi per ml of randomized ³³P-ATP | |

### 4. Identification of the C-atom positions of the glucose molecules of an alpha-1,4-glucan, into which phosphate residues are introduced by a starch-phosphorylating protein

The C-atom positions of the glucose molecules of an alpha-1,4-glucan which are phosphorylated by a protein can be detected by subjecting the glucans obtained, which have been phosphorylated *in vitro* by a particular protein, to hydrolysis, subsequently separating the glucose monomers obtained after the hydrolysis step, followed by measuring the phosphate which has been incorporated by a particular protein into specific fractions of the glucose molecules.

### a) Total hydrolysis of the alpha-1,4-glucans

Aqueous suspensions comprising alpha-1,4-glucan are centrifuged, the sedimented pellet is subsequently resuspended in 0.7 M HCl (Baker, analytical grade) and incubated for 2 hours at 95°C with shaking. After the incubation has ended, the samples are briefly cooled and then centrifuged (for example 2 minutes, 10000xg). The supernatant obtained is transferred into a fresh reaction vessel and neutralized by adding 2 M NaOH (Baker, analytical grade). If a pellet remains, it is resuspended in 100 µl of water, and the amount of the labeled phosphate found therein is determined for control purposes.
The neutralized supernatant is subsequently centrifuged through a 10 kDa filter. By measuring an aliquot of the filtrate obtained, the amount of labeled phosphate in the filtrate is determined, for example with the aid of a scintillation counter.

### b) Fractionation of the hydrolysates, and determination of the phosphorylated C-atom positions

The neutralized hydrolytic filtrates obtained in step a) (approximately 3000 cpm when using radiolabeled ATP) can be separated with the aid of, for example, high-pressure anion exchange chromatography (HPAE). To bring the neutralized filtrate to the volume required for the HPAE, it can be diluted with H₂O. Furthermore, glucose 6-phosphate (approx. 0.15 mM) and glucose 3-phosphate (approx. 0.3 mM) are added to each of the filtrates in question to act as internal controls. The separation by means of HPAE can be effected for example with the aid of a DX 600 Bio Lc system from Dionex using a CarboPac 100 column (equipped with suitable precolumn) and a pulsed amperometric detector (ED 50). Before the sample is injected, the column is first flushed for 10 minutes with 99% eluent C and 1% eluent D. Thereafter, in each case 60 µl of sample volume are injected. The sample is eluted by the following conditions:

| | |
|---|---|
| Flow rate: | 1 ml per minute |
| Gradient: | linear gradient from 0 minutes to 30 minutes |

| | Eluent C | Eluent D |
|---|---|---|
| 0 minutes | 99% | 1% |
| 30 minutes | 0% | 100% |
| 35 minutes | 0% | 100% |
| Run stops | | |

The hydrolysates eluted from the column are collected in individual fractions of in each case 1 ml. Since nonlabeled glucose 3-phosphate (Ritte et al. 2002, PNAS 99, 7166-7171) and nonlabeled glucose 6-phosphate (Sigma, Prod. No.: G7879) had been admixed to each of the injected hydrolysate samples as internal standards, those fractions comprising either glucose 3-phosphate or glucose 6-phosphate can be determined by means of pulsed amperometric detection. By measuring the amount of labeled phosphates in the individual fractions and subsequently comparing them with the fractions which comprise glucose 3-phosphate or glucose 6-phosphate, it is possible to determine those fractions in which labeled glucose 6-phosphate or labeled glucose 3-phosphate is present The amount of labeled phosphate in the fraction in question is determined. It is now possible to determine the C-atom position which is preferentially phosphorylated by an alpha-1,4-glucan-phosphorylating enzyme on the basis of the ratios of the amounts, measured for labeled phosphate, of glucose 3-phosphate versus glucose 6-phosphate in the individual hydrolysates.

### c) Buffers used

| | |
|---|---|
| Eluent C: | 100 mM NaOH |
| Eluent D: | 100 mM NaOH |
| | 500 mM sodium acetate |

### 5. Transformation of rice plants

Rice plants were transformed by the method described by Hiei et al. (1994, Plant Journal 6(2), 271-282).

### 6. Determination of the starch phosphate content

### a) Determination of the C-6-phosphate (C-6-P) content

In starch, the positions C2, C3 and C6 of the glucose units can be phosphorylated. To determine the C6-P content of the starch, 50 mg of starch are hydrolyzed in 500 µl of 0.7 M HCl for 4 hours at 95°C. Thereafter, the mixtures are centrifuged for 10 minutes at 15500xg and the supernatants are removed. 7 µl of the supernatants are mixed with 193 µl of imidazole buffer (100 mM imidazole, pH 7.4; 5 mM MgCl₂, 1 mM EDTA and 0.4 mM NAD). The measurement was carried out in a photometer at 340 nm. After a basic absorption had been established, the enzyme reaction was started by addition of 2 units of glucose-6-phosphate dehydrogenase (from Leuconostoc mesenteroides, Boehringer Mannheim). The change in absorption is directly proportional to the concentration of the G 6-P content of the starch.

### b) Determination of the total phosphate content

The total phosphate content was determined by the method of Ames (Methods in Enzymology VIII, (1966). 115-118).
Approximately 50 mg of starch are created with 30 µl of ethanolic magnesium nitrate solution and the mixture is ashed for three hours at 500°C in the muffle furnace. The residue is treated with 300 µl of 0.5 M hydrochloric acid and incubated for 30 minutes at 60°C. An aliquot portion is subsequently made up to 300 µl of 0.5 M hydrochloric acid, and this is added to a mixture of 100 µl of 10% strength ascorbic acid and 600 µl of 0.42% strength ammonium molybdate in 2 M sulfuric acid and incubated for 20 minutes at 45°C.

### c) Determination of the C-6-phosphate and C-3-phosphate contents

To determine the content of phosphate which is bound in the C-6 position and in the C-3 position of the glucose molecules of an alpha-1,4-glucan, the glucans in question can be subjected to a total hydrolysis and then separated by means of HPAE. The amounts of glucose 6-phosphate and glucose 3-phosphate can be determined by integration of the individual peak areas obtained after HPEA separation. By comparing the resulting peak areas for glucose 6-phosphate and glucose 3-phosphate in unknown samples with the peak areas obtained with known amounts of glucose 6-phosphate and glucose 3-phosphate after separation by means of HPEA, it is possible to determine the amount of glucose 6-phosphate and glucose 3-phosphate in the samples to be tested.

### Examples

### 1.Information on vectors and plasmids

### Generation of the expression vector ME5/6

pGSV71 is a derivative of the plasmid pGSV7, which is derived from the intermediary vector pGSV1. pGSV1 is a derivative of pGSC1700, whose construction has been described by Comelissen and Vanderwiele (Nucleic Acid Research 17, (1989), 19-25). pGSV1 was obtained from pGSC1700 by deleting the carbenicillin resistance gene and deleting the T-DNA sequences of the TL-DNA region of the plasmid pTiB6S3.
pGSV7 contains the replication origin of the plasmid pBR322 (Bolivar et al., Gene 2, (1977), 95-113) and the replication origin of the *Pseudomonas* plasmid pVS1 (Itoh et al., Plasmid 11, (1984), 206). pGSV7 additionally contains the selectable marker gene *aadA*, from the *Klebsiella pneumoniae* transposon Tn1331, which confers resistance to the antibiotics spectinomycin and streptomycin (Tolmasky, Plasmid 24 (3), (1990), 218-226; Tolmasky and Crosa, Plasmid 29(1), (1993), 31-40)
The plasmid pGSV71 was obtained by cloning a chimeric *bar* gene between the border regions of pGSV7. The chimeric *bar* gene contains the cauliflower mosaic virus promoter sequence for transcriptional initiation (Odell et al., Nature 313, (1985), 180), the *Streptomyces hygroscopicus bar* gene (Thompson et al., Embo J. 6, (1987), 2519-2523) and the 3'-untranslated region of the pTiT37 T-DNA nopalin synthase gene for transcriptional termination and for polyadenylation. The *bar* gene confers tolerance to the herbicide glufosinate-ammonium.
The T-DNA contains the right border sequence of the TL-DNA from the plasmid pTiB6S3 (Gielen et al., EMBO J. 3, (1984), 835-846) at position 198-222. A polylinker sequence is located between nucleotide 223-249. The nucleotides 250-1634 contain the cauliflower mosaic virus p35S3 promoter region (Odell et al., see above). The coding sequence of the *Streptomyces hygroscopicus* phosphinothricin resistance gene (*bar*) (Thompson et al. 1987, see above) is arranged between the nucleotides 1635-2186. The two terminal codons at the 5' end of the *bar* wild-type gene were replaced by the codons ATG and GAC. A polylinker sequence is located between the nucleotides 2187-2205. The 260 bp Taql fragment of the untranslated 3' end of the nopalin synthase gene (3'nos) from the T-DNA of the plasmid pTiT37 (Depicker et al., J. Mol. Appl. Genet. 1, (1982), 561-573) is located between the nucleotides 2206 and 2465. The nucleotides 2466-2519 contain a polylinker sequence. The left border sequence of the pTiB6S3 TL-DNA (Gielen et al., EMBO J. 3, (1984), 835-846) is located between the nucleotides 2520-2544.
The vector pGSV71 was then cut using the enzyme PstI and made blunt-ended. The B33 promoter and the ocs cassette was then excised from the vector pB33-Kan in the form of an *Eco*RI*-Hind*III fragment, made blunt-ended by filling in the ends and inserted into the vector pGSV71 which had been cut with *Pst*I and made blunt-ended. The resulting vector (ME4/6) was used as starting vector for the construction of ME5/6: An oligonucleotide containing the cleavage sites *Eco*RI, *Pac*I, *Spe*I*, Srf*I, *Spe*I, *Not*I, *Pac*I and *Eco*RI was introduced into the *Pst*I cleavage site of the vector ME4/6 located between the B33 promoter and the ocs element, duplicating the *Pst*I cleavage site. The resulting expression vector was termed ME5/6.

### Generation of the expression vector pML80

Then, a *BamH*I fragment of ME5-6 was exchanged for a PCR product which had been extended by some cleavage sites, but which was otherwise identical, giving rise to the plasmid pUL1-17. The B33 promoter, which is present in pUL1-17, was excised using the restriction enzymes *Hind*III and *Pst*I, and the vector was made blunt-ended and religated, giving rise to the vector pML18-56. This vector was opened with *Mun*I and *Pst*I, and an MCS (multiple cloning site) which had suitable sticky ends and which had been synthesized by two annealed oligonucleotides (GAG CTC CTA GGC TCG AGT TAA CAC TAG TAA GCT TAA TTA AGA TAT CAT TTA CA and AAT TGT AAA TGA TAT CTT AAT TAA GCT TAC TAG TGT TAA CTC GAG CCT AGG AGC TCT GCA) was inserted. The resulting plasmid pML72-129 was cut with the restriction enzyme *Bam H*I, the overhangs of the cleavage sites were filled up with the aid of Klenow polymerase, and the resulting blunt ends were ligated. This gave rise to a *Cla I* restriction cleavage site. The resulting plasmid named pIR91-123. The ubiquitine promoter from maize was excised from pML8-52 by restriction digestion with the enzymes *Sal I* and *Not I* and made blunt-ended, and the resulting fragment was cloned into the *Eco RV-*cut plasmid pIR91-123. The resulting plasmid was named pML80-123.

### Generation of the vector pML8-52:

pMCS5 (MobiTec, Prod. No.: pMCS5) was cut with Bg*l II* and *BamHI* and religated. The resulting plasmid was named pML4-52. The nos terminator of the *Agrobacterium tumefaciens* nopalin synthase gene (Depicker et al., J. Mol. Appl. Genet. 1, (1982), 561-573), which had been amplified with the aid of the oligonucleotides PML9 (ACT TCT GCA GCG GCC GCG ATC GTT CAA ACA TTT GGC AAT AAA GTT TC) and PML10 (TCT AAG CTT GGC GCC GCT AGC AGA TCT GAT CTA GTA ACA TAG ATG ACA CC) was cloned into this vector and into the restriction cleavage sites *Pst I* and *Hind III.* The resulting plasmid was named pML4-nos. A *Pst I* fragment 1986 base pairs in length and comprising the promoter of the polyubiquitin gene from maize (Genbank Acc.: 94464, Christensen et al., 1992, Plant Mol. Biol. 18: 675-689) and the first intron of the same gene which had been truncated by digestion with *Cla I* and religation was cloned into the aforementioned vector. The resulting plasmid was named pML8-52.

### 2. Isolation of sequences coding for an Arabidopsis thaliana R3 protein

The sequence coding for an *Arabidopis thaliana* R3 protein was isolated by amplification, by means of RT-PCR, of a plurality of overlapping sequences starting from mRNA isolated from leaves of approximately three-week-old *Arabidopsis thaliana* plants (ecotype C24) and subsequent sequential cloning of the amplified fragments. The following primers were used:

| **Name** | **Sequence** |
|---|---|
| **F1** | AAGCTGCCATGGCAACCTCTAAATC |
| **R2** | AAAACTCGAGCTTAAACTTGGGGTCTAGCTTGGA |
| **R4** | CTCAGAGCCATTGGAGCACTCA |
| **F4** | CATTGCAAACAAAAGCGGTCCTTG |

To this end, a cDNA strand was synthesized by means of reverse transcriptase Revert Aid H Minus First Strand cDNA Synthesis Kit (Fermentas Prod. No: EP0451) and subsequently amplified using DNA polymerase (Platinum Taq DNA Polymerase High Fidelity; Invitrogen Prod No.: 11304).

An approx. 3.2 kb DNA fragment was amplified using 300 ng of poly-A-plus RNA with the aid of the primers F1 and R4 under the following reaction conditions:

### First-strand synthesis:

The conditions and buffers specified by the manufacturer were used. The reaction mixture for the first-strand synthesis additionally comprised the following substances:

| | |
|---|---|
| 300 ng | poly-A-plus RNA |
| 0.5 µM | primer R2 |

The reaction mixture was brought to a volume of 12 µl with water, incubated for 5 minutes at 70°C and then cooled on ice.
Thereafter, 5x reaction buffer (4 µl), RNAse inhibitor (1 µl) and dNTP mix (2 µl of 10 mM) were added and the reaction mixture was incubated for 5 minutes at 37°C. After addition of reverse transcriptase (1 µl), cDNA synthesis took place for 60 minutes at 42 C, before the reaction was stopped by heating for 5 minutes at 70°C.

Conditions for the amplification of the 3.2 kb R3 fragment by means of PCR:

| | |
|---|---|
| 2 µl | of the first-strand synthesis reaction mixture |
| 0.6 µM | primer F1 |
| 0.6 µM | primer R4 |
| 0.4 µM | dNTP mix |
| 3 mM | MgSO₄ |
| 1x | reaction buffer |
| 1.25 units | Platinum Taq DNA Polymerase High Fidelity |

Reaction conditions:

| | | |
|---|---|---|
| Step 1 | 95°C | 2 min |
| Step 2 | 95°C | 45 sec |
| Steep 3 | 61.7°C | 45 sec |
| Step 4 | 68°C | 3 min 30 sec + 1 sec/cycle |

Repeat 35 times from step 2
Thereafter 4°C

After the PCR reaction mixture had been separated by means of an 0.8% TBE-agarose gel, the fragment obtained was excised using a surgical blade, and the DNA was isolated by means of gel extraction kit (Quiagen: Prod. No.: 28704). Thereafter, the fragment which had been isolated was cut with the restriction endonucleases *Nco I* and *Eco RI* and cloned into the cloning vector pMCS5 (MobiTec, Prod. No.: pMCS5) which had been cut with the same restriction endonucleases. The resulting plasmid was named pRS79-191. The sequence analysis of the PCR product pRS79-191 which had been cloned into the plasmid RS79-191 revealed that the fragment contained an insertion which resulted in the interruption of the coding sequence. A corresponding comparison with the corresponding sequence from the NCBI database and an analysis of the inserted sequence suggested that the insertion might be an intron which had not been excised. Since it was unclear whether this putative intron can be spliced out in transgenic plants, subsequent cloning steps involved an exchange in this region for a "insertion-free" sequence, whereby the continuous reading frame of the *Arabidopsis thaliana* R3 protein is restored.

To complement the coding sequence of the *Arabidopsis thaliana* R3 protein in pRS79-191, the missing 3' region of the abovementioned coding sequence was amplified under the reaction conditions detailed hereinbelow starting from 300 ng of poly-A-plus RNA with the aid of the primers F4 and R2:
The cDNA was synthesized as described above, except that not the primer R2, but an oligo dT (part of the RT kit) was used.

The amplification was carried out under the following conditions:

| | |
|---|---|
| 2 µl | of the first-strand synthesis reaction mixture |
| 0.3 µM | primer F4 |
| 0.3 µM | primer R2 |
| 0.4 mM | dNTP mix |
| 2 mM | MgSO₄ |
| 1x | reaction buffer |
| 1.25 units | Platinum Taq DNA Polymerase High Fidelity |

### Reaction conditions:

| | | |
|---|---|---|
| Step 1 | 95°C | 2 min |
| Step 2 | 95°C | 30 sec |
| Step 3 | 61°C | 1 min |
| Step 4 | 68°C | 1 min 45 sec |

Repeat 40 times from step 2
Thereafter 4°C

After separation of the PCR mixture by means of a 0.8% TBE agarose gel, the resulting 1.7 kb fragment was excised from the gel and the DNA was isolated by means of gel extraction kit (Qiagen; Prod. No.: 28704). After treatment with the restriction endonucleases *Xho I* and *Spe I*, the plasmid was cloned into the plasmid pRS79-191, which had been cut with the same restriction endonucleases. The resulting plasmid was named pRS80-227.

### 3. Translational fusion of sequences which code for an R3 protein with sequences which code for a plastidic signal peptide

First, the sequence encoding the plastidic signal peptide of the *Oryza sativa* (variety M202) *dull-I* gene was amplified by means of PCR using a cDNA clone (pRS55-132) as template using the primers SP-Mac fwd (5'-CGA TAT CTG CAG GTT TTG GCA ATG GAG AT-3') and SP-Mac-rev (5'-CTT CGA ACA ATT CTG CTT CCA G-3') and cloned into the vector pCR 2.1 (Invitrogen. Prod. No.: K2000-01). The resulting plasmid was named plC328-215. The cDNA clone pRS55-132 was isolated by hybridizing a phage cDNA library, prepared from *Oryza sativa* poly-A-plus RNA, with a radioactive probe comprising sequences coding for a maize *dul I* protein (EMBL Accession: TO1265; Plant Cell 10; 399-412 (1998)).
Using the plasmid plC328-215 as template, the plastidic signal peptide of the *Oryza* sativa *dul I* gene was amplified with the aid of the primers Os_dul-I_SP-Pac (5'-GAC CTT AAT TAA GGT TTT GGC AAT GGA GAT G-3') and Os_dul-I_SP_Asu (5'-GCC AGG TTT CGA ACA ATT CTG CTT CGA-3'). The 5' primer (Os_dul-I_SP-Pac) comprised a *Pac I* restriction cleavage site and the 3' primer (Os_dul-I_SP_Asu) a *Bst BI* restriction cleavage site.

The amplification was carried out under the following conditions:

| | | |
|---|---|---|
| 10 ng | plC328-215 | |
| 0.2 µM | | primer OS_dul1_SP-Pac |
| 0.2 µM | | primer Os_dul1_SP-Asu |
| 0.4 mM | dNTP mix | |
| 3 mM | MgSO₄ | |
| 1x | reaction buffer | |
| 1.25 units | Platinum Taq DNA Polymerase High Fidelity | |

### Reaction conditions:

| | | |
|---|---|---|
| Step 1 | 95°C | 4 min |
| Step 2 | 95°C | 30 sec |
| Step 3 | 60°C | 30 sec |
| Step 4 | 68°C | 20 sec |

Repeat 40 times from step 2
Thereafter 4°C

After digestion of the resulting PCR fragment with the restriction endonucleases *Pac I* and *Bst BI*, the PCR fragment was cloned into the vector pMCS5 (MobiTec, Prod. No.: pMCS5) which had been cut with the same restriction endonucleases. The resulting plasmid was named pAH41-227.
Thereafter, the sequence which codes for the *Arabidopsis thaliana* R3 protein was amplified by means of PCR using the plasmid pRS80-191 as template and, the primers At_R3_Asu (5'-AAT TGT TCG AAA CCT GGC AAC CTC TAA ATC CC-3') and R2 (see above). The sequence of the 5' primer was selected in such a way that the start codon of the sequence which codes for the R3 protein was not present in the sequence amplified by means of PCR and that a *Bst BI* restriction cleavage site was introduced. The sequence of the 3' primer R2 comprised an *Xho I* restriction cleavage site.

The amplification was carried out under the following conditions:

| | |
|---|---|
| 20 ng | pRS80-227 |
| 0.3 µM | primer At_R3_Asu |
| 0.3 µM | primer R2 |
| 0.4 mM | dNTP mix |
| 3 mM | MgSO₄ |
| 1x | reaction buffer |
| 1.25 units | Platinum Taq DNA Polymerase High Fidelity |

### Reaction conditions:

| | | |
|---|---|---|
| Step 1 | 95°C | 2 min |
| Step 2 | 95°C | 50 sec |
| Step 3 | 60°C | 50 sec |
| Step 4 | 68°C | 4 min |

Repeat 40 times from step 2
Thereafter 4°C

The resulting PCR product was cut with the restriction endonucleases *Bst BI* and *Xho I* and cloned into the plasmid pAH41-227 which had been cut with *Bst BI* and *Sal I,* which gave rise to the plasmid AH42-227. Thus, a sequence was obtained in which the plastidic signal peptide of the *dul I* gene from *Oryza sativa* and the coding region of the *Arabidopsis thaliana* R3 protein form a shared open reading frame, so that, after transcription and translation, a translationally fused R3 protein which has a plastidic signal peptide is obtained.

### 4. Generation of the plant expression vector pAH43-227

The sequence coding for the *Arabidopsis thaliana* R3 protein which was fused to the sequence coding for the plastidic signal peptide of the *Oryza sativa dul I* gene was excised from the plasmid pAH42-227 with the aid of the restriction endonucleases *Pac I* and *Avr II* and cloned into the plasmid pML80-123 which had been cut with the same restriction nucleases.

### 5. Generation of the plant expression vector pIR138-210 in which noncoding sequences were removed from the coding region of the R3 protein

The fragment which is present within the coding region of the *Arabidopsis thaliana* R3 protein and which comprises noncoding sequences was removed from the plasmid pAH43-227.
To this end, a part-sequence of the coding region of the *Arabidopsis thaliana* R3 protein was amplified by means of RT-PCR starting from mRNA isolated from leaves of approximately three-week old *Arabidopsis thaliana* plants (ecotype C24).

The following primers were used:

| **Name** | **Sequence** |
|---|---|
| **F1** | AAGCTGCCATGGCAACCTCTAAATC |
| **R5** | CCAAGATATTTTGCAGTAGGCTGT |

The first-strand synthesis was carried out as described above (Example, Item 2).
The desired fragment was amplified under the following conditions:

| | |
|---|---|
| 2 µl | of the first-strand synthesis reaction mixture |
| 0.3 µM. | primer F1 |
| 0.3 µM | primer R5 |
| 0.4 mM | dNTP mix |
| 3 mM | MgSO₄ |
| 1x | reaction buffer |
| 1.25 units | Platinum Taq DNA Polymerase High Fidelity |

Reaction conditions:

| | | |
|---|---|---|
| Step 1 | 95°C | 2 min |
| Step 2 | 95°C | 1 min |
| Step 3 | 61°C | 1 min |
| Step 4 | 68°C | 2 min 20 sec |

Repeat 40 times from step 2
Thereafter 4°C

Since the insertion present in the plasmids pRS79-191, pRS80-227, AH42-227 and AH43-227 (putative intron) has an *Ssp I* restriction cleavage site, the part sequence of the coding region of the *Arabidopsis thaliana* R3 protein, which has been amplified by means of PCR, was first digested with the restriction endonuclease *Ssp I* and undigested fragments (without insertion) were subsequently purified by means of an agarose gel. Thereafter, the purified fragment was cut with the restriction endonucleases *Bam HI* and *Spe I* and exchanged for the corresponding *Bam HI*/*Spe I* fragment (comprising, inter alia the noncoding sequences) of the plasmid pAH43-227. The resulting plasmid was named pIR138-210.

### 6. Generation of the plant expression vector pIR139-210 in which the coding sequence of the R3 protein has no plastidic signal peptide

Using the plasmid pIR 138-210 as template, a part-sequence coding for the 5' region of the *Arabidopsis thaliana* R3 protein was amplified by means of PCR with the aid of the primers IR138-F1 (5'-AAT TAA CTT AAT TAA TCC ATG GCA ACC TCT AAA TCC CAA CAA T-3') and IR138-R2 (5'-AAT TAC TTG GAT CCC GCA ACT CAA GGA TCA CTA CAT ATG CA-3'). The 5' primer (IR138-F1) comprised a *Pac I* restriction cleavage site and the sequence for the original start codon of the R3 protein. The 3' primer (IR138-R2) comprised the recognition sequence of the restriction endonuclease *BamHI.*
The amplification was carried out under the following conditions:

| | |
|---|---|
| 50 ng | plasmid IR138-210 |
| 0.5 µM | primer IR138-F1 |
| 0.5 µM | primer IR138-R2 |
| 2 mM | MgSO₄ |
| 0.3 mM | dNTP mix |
| 1 x | reaction buffer |
| 1.5 units | Platinum Taq DNA Polymerase High Fidelity |

Reaction conditions:

| | | |
|---|---|---|
| Step 1 | 95°C | 2 min |
| Step 2 | 94°C | 30 sec |
| Step 3 | 60°C | 20 sec |
| Step 4 | 68°C | 20 sec |

Repeat 30 times from step 2
Thereafter 4°C

The amplified 247 bp fragment was cut with the restriction endonucleases *Pac I* and *Bam HI* and replaced by the *Pac I*/*Bam H I* fragment which is present in the plasmid pIR138-210 and which comprises the plastidic signal peptide of the *dull I* gene from *Oryza sativa* and part of the 5' sequence which codes for the *Arabidopsis thaliana* R3 protein. The resulting plasmid was named pIR139-210. The coding sequence of the *Arabidopsis thaliana* R3 protein comprises the start codon which is originally from the cDNA, in contrast to the sequence comprising in the plasmid pIR138-210 and AH43-227.

### 7. Transformation of rice plants

Rice plants (variety M202) were transformed with the plasmid pIR138-210 or with the plasmid pIR139-210 by means of agrobacterium (comprising the plasmid pIR138-210 or pIR139-210) using the method described by Hiei et al. (1994, Plant Journal 6(2), 271-282).

### 8. Analysis of the transgenic rice plants

### a) Rice plants which had been transformed with the plasmid pIR138-210 and which expressed an Arabidopsis thaliana R3 protein in fusion with a plastidic signal peptide

Quantitative RT-PCR analysis was used for the identification of plants with expression of mRNA coding for the *Arabidopsis thaliana* R3 protein.
Plants which, in comparison with corresponding wild-type plants, comprise a detectable amount of mRNA coding for the *Arabidopsis thaliana* R3 protein were grown in the greenhouse. Grains from these plants were harvested. Starch isolated from these mature grains showed an increased content in phosphate which was covalently bonded to the starch in question in comparison with starch isolated from grains of corresponding wild-type plants.

### b) Rice plants which were transformed with the plasmid pIR139-210 and expressed an Arabidopsis thaliana R3 protein

Using quantitative RT-PCR analysis, it was possible to identify plants which express mRNA coding for the *Arabidopsis thaliana* R3 protein.
Plants which, in comparison with corresponding wild-type plants, comprise a detectable amount of mRNA coding for the *Arabidopsis thaliana* R3 protein were grown in the greenhouse. Grains from these plants were harvested. Starch isolated from these mature grains showed no significantly increased content in phosphate which was covalently bonded to the starch in question in comparison with starch isolated from grains of corresponding wild-type plants.

## Claims

1. A genetically modified plant cell wherein plastids present in said plant cell comprise a protein which transfers a phosphate residue from a nucleoside triphosphate to starch, wherein the genetic modification consists in the introduction of a foreign nucleic acid molecule into the genome of the plant cell, where the foreign nucleic acid molecule is selected from the group consisting of
a) nucleic acid molecules which code for a protein with the amino acid sequence shown in SEQ ID NO 3,
b) nucleic acid molecules which code for a protein which comprises the amino acid sequence which is encoded by the insertion in plasmid DSM 16587 or by the insertion in plasmid DSM 16645;
c) nucleic acid molecules which code for a protein whose sequence has at least 60% identity with the amino acid sequence shown in SEQ ID NO 3;
d) nucleic acid molecules which code for a protein whose sequence has at least 60% identity with the amino acid sequence which is encoded by the insertion in plasmid DSM 16587 or by the insertion in plasmid DSM 16645;
e) nucleic acid molecules which comprise the nucleotide sequence shown in SEQ ID NO 1 or SEQ ID NO 2 or a complementary sequence;
f) nucleic acid molecules which comprise the nucleotide sequence of the insertion present in plasmid DSM 16587 or in plasmid DSM 16645;
g) nucleic acid molecules which have at least 60% identity with the nucleic acid sequences described in a), b), e) or f);
h) nucleic acid molecules which hybridize, under stringent conditions with at least one strand of the nucleic acid molecules described in a), b), e) or f);
i) nucleic acid molecules whose nucleotide sequence, as the result of the degeneracy of the genetic code, deviates from the sequence of the nucleic acid molecules mentioned in a), b), e) or f); and
j) nucleic acid molecules which constitute fragments, allelic variants and/or derivatives of the nucleic acid molecules mentioned in a), b), c), d), e), f), g), h) or i)
and wherein the foreign nucleic acid molecule is a recombinant nucleic acid molecule comprising additional sequences which are not naturally present in a combination in which they are present in the recombinant nucleic acids where the nucleic acid molecule encoding the protein which transfers a phosphate residue from a nucleoside triphosphate to starch is fused to nucleic acid sequences which code for a plastidic signal peptide so that the recombinant nucleic acid molecule codes for a protein which transfers a phosphate residue from a nucleoside triphosphate to starch and which in addition to its coding amino acid sequence has a plastidic signal sequence.

2. The plant cell as claimed in claim 1 which synthesizes a modified starch in comparison with corresponding non-genetically-modified wild-type plant cells, wherein the modified starch comprises an increased starch phosphate content and/or a modified phosphate distribution.

3. A plant which comprises genetically modified plant cells as claimed in any of claims 1 and 2.

4. The plant as claimed in claim 3, which is a starch-storing plant.

5. Propagation material of a plant as claimed in claim 3 or 4, comprising a plant cell as claimed in any of claims 1 or 2.

6. A harvestable plant part of a plant as claimed in claim 3 or 4, comprising a plant cell as claimed in any of claims 1 or 2.

7. A method for generating a genetically modified plant, wherein
a) a plant cell is transfromed, the transformation leading to an increase in the activity of at least one protein coding a protein which transfers a phosphate residue from a nucleoside triphosphate to starch in comparison with corresponding non-genetically-modified wild-type plant cells and consists in the introduction of a foreign nucleic acid molecule coding a protein which transfers a phosphate residue from a nucleoside triphosphate to starch selected from the group consisting of I
i) nucleic acid molecules which code for a protein with the amino acid sequence shown in SEQ ID NO 3;
ii) nucleic acid molecules which code for a protein which comprises the amino acid sequence which is encoded by the insertion in DSM 16587 or by the insertion in DSM 16645;
iii) nucleic acid molecules which code for a protein whose amino acid sequence has at least 60% identity with the amino acid sequence shown in SEQ ID NO 3;
iv) nucleic acid molecules which code for a protein whose sequence has at least 60% identity with the amino acid sequence which is encoded by the insertion in plasmid DSM 16587 or by the insertion in plasmid DSM 16645;
v) nucleic acid molecules which comprise the nucleotide sequence shown in SEQ ID NO 1 or SEQ ID NO 2 or a complementary sequence;
vi) nucleic acid molecules which comprise the nucleotide sequence of the insertion present in plasmid DSM 16587 or in plasmid DSM 16645;
vii) nucleic acid molecules which have at least 70% identity with the nucleic acid sequences described in i), ii), v) or vi);
viii) nucleic acid molecules which hybridize under stringent conditions with at least one strand of the nucleic acid molecules described in i), ii), v) or vi);
iix) nucleic acid molecules whose nucleotide sequence, as the result of the degeneracy of the genetic code, deviates from the sequence of the nucleic acid molecules mentioned in i), ii), v) or vi); and
ix) nucleic acid molecules which constitute fragments, allelic variants and/or derivatives of the nucleic acid molecules mentioned in i), ii), iii), iv), v), vi), vii), viii) or iix),
into the genome of the plant cell, wherein said foreign nucleic acid molecule is a recombinant nucleic acid molecule comprising additional sequences which are not naturally present in a combination in which they are present in the recombinant nucleic acids in which a nucleic acid sequence encoding a protein which transfers a phosphate residue from a nucleoside triphosphate to starch is fused with a nucleic acid sequence which codes for a plastidic signal peptide so that the recombinant nucleic acid molecule codes for a protein which transfers a phosphate residue from a nucleoside triphosphate to starch and which in addition to its coding amino acid sequence has a plastidic signal peptide,
b) a plant is regenerated from plant cells of step a); and
c) if appropriate, further plants are generated with the aid of the plants of step b).

8. A method for producing a modified starch, comprising the step of extracting the starch from a plant cell as claimed in any of claims 1 or 2 or extracting the starch from a plant as claimed in claim 3 or 4.

9. A method for producing a modified starch, comprising the step of extracting the starch from harvestable plant parts as claimed in claim 6.

10. A method for producing flours comprising the step of grinding parts of plants as claimed in claim 3 or 4 or of propagation material as claimed in claim 5 or harvestable material as claimed in claim 6.

11. The use of a genetically modified plant cell as claimed in any of claims 1 or 2 or of a plant as claimed in claim 3 or 4 for producing a flour.

12. A nucleic acid molecule coding for a protein with the enzymatic activity of a protein which transfers a phosphate residue from a nucleoside triphosphate to starch, selected from the group consisting of
a) a nucleic acid molecule which codes for a protein with the amino acid sequence shown in SEQ ID NO 3;
b) a nucleic acid molecule which codes for a protein whose amino acid sequence has at least 60% identity with the amino acid sequence shown in SEQ ID NO 3;
c) a nucleic acid molecule which comprises the nucleotide sequence shown in SEQ ID No. 1 or SEQ ID NO 2 or a sequence which is complementary to these sequences;
d) a nucleic acid molecule which has at least 70% identity with the nucleic acid sequences described in a) or c);
e) a nucleic acid molecule which hybridizes under stringent conditions with at least one strand of the nucleic acid molecule described in a) or c);
f) a nucleic acid molecule whose nucleotide sequence, as the result of the degeneracy of the genetic code, deviates from the sequence of the nucleic acid molecules mentioned in a) or c); and
g) a nucleic acid molecule which constitutes a fragment, allelic variant and/or derivative of the nucleic acid molecules mentioned in a), b), c), d), e) or f),
and wherein said nucleic acid molecule is a recombinant nucleic acid molecule comprising additional sequences which are not naturally present in a combination in which they are present in the recombinant nucleic acid, where the nucleic acid molecule encoding a protein which transfers a phosphate residue from a nucleoside triphosphate to starch is fused to nucleic acid sequences which code for a plastidic signal peptide.

13. A vector comprising a nucleic acid molecule as claimed in claim 12.

14. The vector as claimed in claim 13, wherein the nucleic acid molecule is linked with regulatory sequences which initiate the transcription in prokaryotic or eukaryotic cells.

15. A host cell which comprises a nucleic acid molecule as claimed in claim 12, or a vector as claimed in claim 13 or 14.

16. A composition comprising a nucleic acid molecule as claimed in claim 12 or a vector as claimed in claim 13 or 14.

17. The use of a composition as claimed in claim 16 for identifying plant cells which, in comparison with non-genetically-modified wild-type plant cells show an increased activity of a protein which transfers a phosphate residue from a nucleoside triphosphate to starch.

18. A protein with starch phosphorylating activity selected from the group consisting of
a) a protein which comprises the amino acid sequence shown in SEQ ID NO 3;
b) a protein which has at least 60% identity with the amino acid sequence of the proteins mentioned in a), fused to amino acid sequences which code for a plastidic signal peptide.

19. A recombinant protein which comprises amino acid sequences coding for a protein according to claim 18 fused to amino acid sequences which code for a plastidic signal peptide.

20. The protein as claimed in claim 18 or 19, which has a phosphohistidine domain.

## Patentansprüche

1. Genetisch modifizierte Pflanzenzelle, wobei Plastiden, die in dieser Pflanzenzelle vorliegen, ein Protein umfassen, das einen Phosphatrest von einem Nukleosidtriphosphat auf Stärke überträgt, wobei die genetische Modifikation in der Einführung eines heterologen Nukleinsäuremoleküls in das Genom der Pflanzenzelle besteht, wobei das heterologe Nukleinsäuremolekül ausgewählt ist aus der Gruppe umfassend
a) Nukleinsäuremoleküle, die für ein Protein mit der Aminosäuresequenz gemäß SEQ ID Nr.3 codieren;
b) Nukleinsäuremoleküle, die für ein Protein codieren, das die Aminosäuresequenz umfasst, die durch die Insertion in Plasmid DSM 16587 oder durch die Insertion in Plasmid DSM 16645 codiert wird;
c) Nukleinsäuremoleküle, die für ein Protein codieren, dessen Sequenz mindestens 60% Identität mit der Aminosäuresequenz gemäß SEQ ID Nr.3 aufweist;
d) Nukleinsäuremoleküle, die für ein Protein codieren, dessen Sequenz mindestens 60% Identität mit der Aminosäuresequenz, die durch die Insertion in Plasmid DSM 16587 oder durch die Insertion in Plasmid DSM 16645 codiert wird, aufweist;
e) Nukleinsäuremoleküle, die die in SEQ ID Nr.1 oder SEQ ID Nr.2 gezeigte Nukleotidsequenz oder eine komplementäre Sequenz umfassen;
f) Nukleinsäuremoleküle, die die Nukleotidsequenz der in Plasmid DSM 16587 oder in Plasmid DSM 16645 vorliegenden Insertion umfassen;
g) Nukleinsäuremoleküle mit mindestens 60% Identität mit den in a), b), e) oder f) beschriebenen Nukleinsäuresequenzen;
h) Nukleinsäuremoleküle, die unter stringenten Bedingungen mit mindestens einem Strang der in a), b), e) oder f) beschriebenen Nukleinsäuremoleküle hybridisieren;
i) Nukleinsäuremoleküle, deren Nukleotidsequenz aufgrund der Degeneration des genetischen Codes von der Sequenz der in a), b), e) oder f) erwähnten Nukleinsäuremoleküle abweicht; und
j) Nukleinsäuremoleküle, die Fragmente, Allelvarianten, und/oder Derivate den in a), b), c), d), e), f), g), h) oder i) erwähnten Nukleinsäuremoleküle darstellen;
und wobei das heterologe Nukleinsäuremolekül ein rekombinantes Nukleinsäuremolekül ist, das zusätzliche Sequenzen umfasst, die nicht natürlich vorliegen in einer Kombination, in der sie in den rekombinanten Nukleinsäuren vorliegen, wobei das für das Protein, das einen Phosphatrest von Nukleosidtriphosphat auf Stärke überträgt, codierende Nukleinsäuremolekül fusioniert ist mit Nukleinsäuresequenzen, die für ein plastidäres Signalpeptid codieren, so dass das rekombinante Nukleinsäuremolekül für ein Protein codiert, das einen Phosphatrest von einem Nukleosidtriphosphat auf Stärke überträgt, und das zusätzlich zu seiner Aminosäure-Codiersequenz eine plastidäre Signalsequenz aufweist.

2. Pflanzenzelle nach Anspruch 1, die im Vergleich zu entsprechenden nichtgenetisch modifizierten Wildtyp-Pflanzenzellen eine modifizierte Stärke synthetisiert, wobei die modifizierte Stärke einen erhöhten Stärkephosphatgehalt und/oder eine modifizierte Phosphatverteilung umfasst.

3. Pflanze, umfassend genetisch modifizierte Pflanzenzellen nach einem der Ansprüche 1 und 2.

4. Pflanze nach Anspruch 3, bei der es sich um eine stärkespeichernde Pflanze handelt.

5. Vermehrungsmaterial einer Pflanze nach Anspruch 3 oder 4, umfassend eine Pflanzenzelle nach einem der Ansprüche 1 oder 2.

6. Erntbarer Pflanzenteil einer Pflanze nach Anspruch 3 oder 4, umfassend eine Pflanzenzelle nach einem der Ansprüche 1 oder 2.

7. Verfahren zur Herstellung einer genetisch modifizierten Pflanze, umfassend die Schritte:
a) Transformieren einer Pflanzenzelle, wobei die Transformation im Vergleich zu entsprechenden nichtgenetisch modifizierten Wildtyp-Pflanzenzellen zu einer Erhöhung der Aktivität von mindestens einem Protein führt, codierend für ein Protein, das einen Phosphatrest von einem Nukleosidtriphosphat auf Stärke überträgt, und darin besteht, dass man ein heterologes Nukleinsäuremolekül, das ein Protein codiert, das einen Phosphatrest von einem Nukleosidtriphosphat auf Stärke überträgt, ausgewählt aus der Gruppe bestehend aus:
i) Nukleinsäuremolekülen, die für ein Protein mit der Aminosäuresequenz gemäß SEQ ID Nr.3 codieren;
ii) Nukleinsäuremolekülen, die für ein Protein codieren, das die Aminosäuresequenz umfasst, die durch die Insertion in DSM 16587 oder durch die Insertion in DSM 16645 codiert wird;
iii) Nukleinsäuremolekülen, die für ein Protein codieren, dessen Aminosäuresequenz mindestens 60% Identität mit der Aminosäuresequenz gemäß SEQ ID Nr.3 aufweist;
iv) Nukleinsäuremolekülen, die für ein Protein codieren, dessen Sequenz mindestens 60% Identität mit der Aminosäuresequenz, die durch die Insertion in Plasmid DSM 16587 oder durch die Insertion in Plasmid DSM 16645 codiert wird, aufweist;
v) Nukleinsäuremolekülen, die die in SEQ ID Nr.1 oder SEQ ID Nr.2 gezeigte Nukleotidsequenz oder eine komplementäre Sequenz umfassen;
vi) Nukleinsäuremolekülen, die die Nukleotidsequenz der in Plasmid DSM 16587 oder in Plasmid DSM 16645 vorliegenden Insertion umfassen;
vii) Nukleinsäuremolekülen mit mindestens 70% Identität mit den in i), ii), v) oder vi) beschriebenen Nukleinsäuresequenzen;
viii) Nukleinsäuremolekülen, die unter stringenten Bedingungen mit mindestens einem Strang der in i), ii), v) oder vi) beschriebenen Nukleinsäuremoleküle hybridisieren;
iix) Nukleinsäuremolekülen, deren Nukleotidsequenz aufgrund der Degeneration des genetischen Codes von der Sequenz der in i), ii), v) oder vi) erwähnten Nukleinsäuremoleküle abweicht; und
ix) Nukleinsäuremoleküle, die Fragmente, Allelvarianten, und/oder Derivate den in i), ii), iii), iv), v), vi), vii), viii) oder ix) erwähnten Nukleinsäuremoleküle darstellen;
in das Genom der Pflanzenzelle einführt, wobei es sich bei dem heterologen Nukleinsäuremolekül um ein rekombinantes Nukleinsäuremolekül handelt, das zusätzliche Sequenzen umfasst, die nicht natürlich vorliegen in einer Kombination, in der sie in den rekombinanten Nukleinsäuren vorliegen, in denen eine für das Protein, das einen Phosphatrest von einem Nukleosidtriphosphat auf Stärke überträgt, codierende Nukleinsäuresequenz fusioniert ist mit einer Nukleinsäuresequenz, die für ein plastidäres Signalpeptid codiert, so dass das rekombinante Nukleinsäuremolekül für ein Protein codiert, das einen Phosphatrest von einem Nukleosidtriphosphat auf Stärke überträgt, und das zusätzlich zu seiner Aminosäure-Codiersequenz ein plastidäres Signalpeptid aufweist,
b) Regenerieren einer Pflanze aus Pflanzenzellen von Schritt a); und
c) gegebenenfalls Erzeugen von weiteren Pflanzen mit Hilfe der Pflanzen von Schritt b).

8. Verfahren zur Herstellung einer modifizierten Stärke, umfassend den Schritt, dass man aus einer Pflanzenzelle nach einem der Ansprüche 1 oder 2 die Stärke extrahiert oder von einer Pflanze nach Anspruch 3 oder 4 die Stärke extrahiert.

9. Verfahren zur Herstellung einer modifizierten Stärke, umfassend den Schritt, dass man aus erntbaren Pflanzenteilen nach Anspruch 6 die Stärke extrahiert.

10. Verfahren zur Herstellung von Mehlen, umfassend den Schritt, dass man Teile von Pflanzen nach Anspruch 3 oder 4 oder Vermehrungsmaterial nach Anspruch 5 oder erntbares Material nach Anspruch 6 mahlt.

11. Verwendung einer genetisch modifizierten Pflanzenzelle nach einem der Ansprüche 1 oder 2 oder einer Pflanze nach Anspruch 3 oder 4 für die Herstellung eines Mehls.

12. Nukleinsäuremolekül, codierend für ein Protein mit der enzymatischen Aktivität eines Proteins, das einen Phosphatrest von einem Nukleosidtriphosphat auf Stärke überträgt, ausgewählt aus der Gruppe bestehend aus
a) einem Nukleinsäuremolekül, das für ein Protein mit der Aminosäuresequenz gemäß SEQ ID Nr.3 codiert;
b) einem Nukleinsäuremolekül, das für ein Protein codiert, dessen Aminosäuresequenz mindestens 60% Identität mit der Aminosäuresequenz gemäß SEQ ID Nr.3 aufweist;
c) einem Nukleinsäuremolekül, das die in SEQ ID Nr.1 oder SEQ ID Nr.2 gezeigte Nukleotidsequenz oder eine zu diesen Sequenzen komplementäre Sequenz umfasst;
d) einem Nukleinsäuremolekül, das mindestens 70% Identität mit den in a) oder c) beschriebenen Nukleinsäuresequenzen aufweist;
e) einem Nukleinsäuremolekül, das unter stringenten Bedingungen mit mindestens einem Strang des in a) oder c) beschriebenen Nukleinsäuremoleküls hybridisiert;
f) einem Nukleinsäuremolekül, dessen Nukleotidsequenz aufgrund der Degeneration des genetischen Codes von der Sequenz der in a) oder c) erwähnten Nukleinsäuremoleküle abweicht;
g) einem Nukleinsäuremolekül, das ein Fragment, eine Allelvariante und/oder ein Derivat der in a), b), c), d), e) oder f) erwähnten Nukleinsäuremoleküle darstellt;
und wobei das Nukleinsäuremolekül ein rekombinantes Nukleinsäuremolekül ist, das zusätzliche Sequenzen umfasst, die nicht natürlich vorliegen in einer Kombination, in der sie in den rekombinanten Nukleinsäuren vorliegen, wobei das für ein Protein, das einen Phosphatrest von einem Nukleosidtriphosphat auf Stärke überträgt, codierende Nukleinsäuremolekül fusioniert ist mit Nukleinsäuresequenzen, die für ein plastidäres Signalpeptid codieren.

13. Vektor, umfassend ein Nukleinsäuremolekül nach Anspruch 12.

14. Vektor nach Anspruch 13, wobei das Nukleinsäuremolekül mit Regulationssequenzen, die die Transkription in prokaryontischen oder eukaryontischen Zellen initiieren, verknüpft ist.

15. Wirtszelle, umfassend ein Nukleinsäuremolekül nach Anspruch 12 oder einen Vektor nach Anspruch 13 oder 14.

16. Zusammensetzung, umfassend ein Nukleinsäuremolekül nach Anspruch 12 oder einen Vektor nach Anspruch 13 oder 14.

17. Verwendung einer Zusammensetzung nach Anspruch 16 zum Identifizieren von Pflanzenzellen, die im Vergleich zu nicht genetisch modifizierten Wildtyp-Pflanzenzellen eine erhöhte Aktivität eines Proteins, das einen Phosphatrest von einem Nukleosidtriphosphat auf Stärke überträgt, aufweisen.

18. Protein mit stärkephosphorylierender Aktivität, ausgewählt aus der Gruppe bestehend aus
a) einem Protein, umfassend die Aminosäuresequenz gemäß SEQ ID Nr. 3;
b) einem Protein mit mindestens 60% Identität zu der Aminosäuresequenz der in a) erwähnten Proteine in Fusion mit Aminosäuresequenzen, die für ein plastidäres Signalpeptid codieren.

19. Rekombinantes Protein, umfassend Aminosäuresequenzen, codierend für ein Protein nach Anspruch 18 in Fusion mit Aminosäuresequenzen, die für ein plastidäres Signalpeptid codieren.

20. Protein nach Anspruch 18 oder 19, das eine Phosphohistidindomäne aufweist.

## Revendications

1. Cellule végétale génétiquement modifiée, dans laquelle les plasmides présents dans ladite cellule végétale comprennent une protéine qui transfère un résidu phosphate d'un nucléoside triphosphate à l'amidon, la modification génétique consistant en l'introduction d'une molécule d'acide nucléique étranger dans le génome de la cellule végétale, la molécule d'acide nucléique étranger étant choisie dans le groupe consistant en
a) les molécules d'acide nucléique qui codent pour une protéine ayant la séquence d'acides aminés présentée dans SEQ ID N° 3 ;
b) les molécules d'acide nucléique qui codent pour une protéine qui comprend la séquence d'acides aminés qui est codée par l'insertion dans le plasmide DSM 16587 ou par l'insertion dans le plasmide DSM 16645 ;
c) les molécules d'acide nucléique qui codent pour une protéine dont la séquence a une identité d'au moins 60 % avec la séquence d'acides aminés présentée dans SEQ ID N°3;
d) les molécules d'acide nucléique qui codent pour une protéine dont la séquence a une identité d'au moins 60 % avec la séquence d'acides aminés qui est codée par l'insertion dans le plasmide DSM 16587 ou par l'insertion dans le plasmide DSM 16645 ;
e) les molécules d'acide nucléique qui comprennent la séquence nucléotidique présentée dans SEQ ID N° 1 ou SEQ ID N° 2 ou une séquence complémentaire ;
f) les molécules d'acide nucléique qui comprennent la séquence nucléotidique de l'insertion présente dans le plasmide DSM 16587 ou dans le plasmide DSM 16645 ;
g) les molécules d'acide nucléique qui ont une identité d'au moins 60 % avec les séquences d'acides nucléiques décrites dans a), b), e) ou f) ;
h) les molécules d'acide nucléique qui, dans des conditions stringentes, s'hybrident à au moins un brin des molécules d'acide nucléique décrites en a), b), e) ou f) ;
i) les molécules d'acide nucléique dont la séquence nucléotidique, en conséquence de la dégénérescence du code génétique, s'écarte de la séquence des molécules d'acide nucléique mentionnées en a), b), e) ou f) ; et
j) les molécules d'acide nucléique qui constituent des fragments, des variants alléliques et/ou des dérivés des molécules d'acide nucléique mentionnées en a), b), c), d), e), f), g), h) ou i),
la molécule d'acide nucléique étranger étant une molécule d'acide nucléique recombinant comprenant des séquences additionnelles qui ne sont pas naturellement présentes dans une combinaison dans laquelle elles sont présentes dans des acides nucléiques recombinants, la molécule d'acide nucléique codant pour la protéine qui transfère un résidu phosphate d'un nucléoside triphosphate à l'amidon étant fusionnée à des séquences d'acides nucléiques qui codent pour un peptide signal plasmidique de telle sorte que la molécule d'acide nucléique recombinant code pour une protéine qui transfère un résidu phosphate d'un nucléoside triphosphate à l'amidon et qui, outre sa séquence d'acides aminés codante, possède une séquence signal plasmidique.

2. Cellule végétale selon la revendication 1, qui synthétise un amidon modifié par comparaison avec des cellules végétales de type sauvage correspondantes n'ayant pas subi de modification génétique, l'amidon modifié comprenant une teneur accrue en phosphate d'amidon et/ou une distribution modifiée des phosphates.

3. Plante qui comprend des cellules végétales génétiquement modifiées selon l'une quelconque des revendications 1 et 2.

4. Plante selon la revendication 3, qui est une plante amylacée.

5. Matériel de reproduction d'une plante selon la revendication 3 ou 4, comprenant une cellule végétale selon l'une quelconque des revendications 1 ou 2.

6. Partie de plante exploitable d'une plante selon la revendication 3 ou 4, comprenant une cellule végétale selon l'une quelconque des revendications 1 ou 2.

7. Procédé de production d'une plante génétiquement modifiée, dans lequel:
a) on transforme une cellule végétale, la transformation conduisant à une augmentation de l'activité d'au moins une protéine codant pour une protéine qui transfère un résidu phosphate d'un nucléoside triphosphate à l'amidon par comparaison avec des cellules végétales de type sauvage correspondantes n'ayant pas subi de modification génétique, et consistant en l'introduction d'une molécule d'acide nucléique étranger codant pour une protéine qui transfère un résidu phosphate d'un nucléoside triphosphate à l'amidon, choisie dans le groupe consistant en :
i) les molécules d'acide nucléique qui codent pour une protéine ayant la séquence d'acides aminés présentée dans SEQ ID N° 3 ;
ii) les molécules d'acide nucléique qui codent pour une protéine qui comprend la séquence d'acides aminés qui est codée par l'insertion dans DSM 16587 ou par l'insertion dans DSM 16645 ;
iii) les molécules d'acide nucléique qui codent pour une protéine dont la séquence d'acides aminés a une identité d'au moins 60 % avec la séquence d'acides aminés présentée dans SEQ ID N° 3 ;
iv) les molécules d'acide nucléique qui codent pour une protéine dont la séquence a une identité d'au moins 60 % avec la séquence d'acides aminés qui est codée par l'insertion dans le plasmide DSM 16587 ou par l'insertion dans le plasmide DSM 16645 ;
v) les molécules d'acide nucléique qui comprennent la séquence nucléotidique présentée dans SEQ ID N° 1 ou SEQ ID N° 2, ou une séquence complémentaire;
vi) les molécules d'acide nucléique qui comprennent la séquence nucléotidique de l'insertion présente dans le plasmide DSM 16587 ou dans le plasmide DSM 16645 ;
vii) les molécules d'acide nucléique qui ont une identité d'au moins 70 % avec les séquences d'acides nucléiques décrites en i), ii), v) ou vi) ;
viii) les molécules d'acide nucléique qui s'hybrident dans des conditions stringentes à au moins un brin des molécules d'acide nucléique décrites en i), ii), v) ou vi);
iix) les molécules d'acide nucléique dont la séquence nucléotidique, en conséquence de la dégénérescence du code génétique, s'écarte de la séquence des molécules d'acide nucléique mentionnées en i), ii), v) ou vi) ; et
ix) les molécules d'acide nucléique qui constituent des fragments, des variants alléliques et/ou des dérivés des molécules d'acide nucléique mentionnées en i), ii), iii), iv), v), vi), vii), viii) ou iix),
dans le génome d'une cellule végétale, ladite molécule d'acide nucléique étranger étant une molécule d'acide nucléique recombinant comprenant des séquences additionnelles qui ne sont pas naturellement présentes dans une combinaison dans laquelle elles sont présentes dans des acides nucléiques recombinants dans lesquels une séquence d'acides nucléiques codant pour une protéine qui transfère un résidu phosphate d'un nucléoside triphosphate à l'amidon est fusionnée à une séquence d'acides aminés qui code pour un peptide signal plastidique de telle sorte que la molécule d'acide nucléique recombinant code pour une protéine qui transfère un résidu phosphate d'un nucléoside triphosphate à l'amidon, qui outre sa séquence d'acides aminés possède un peptide signal plastidique,
b) on régénère une plante à partir des cellules végétales de l'étape a) ; et
c) si cela est approprié, on produit des plantes supplémentaires à l'aide des plantes de l'étape b).

8. Procédé de production d'un amidon modifié, qui comprend l'étape d'extraction de l'amidon à partir d'une cellule végétale selon l'une quelconque des revendications 1 ou 2 ou l'extraction de l'amidon à partir d'une plante selon la revendication 3 ou 4.

9. Procédé de production d'un amidon modifié, qui comprend l'étape d'extraction de l'amidon à partir des parties de plante exploitables selon la revendication 6.

10. Procédé de production de farines, qui comprend l'étape de mouture de parties de plante selon la revendication 3 ou 4 ou d'un matériel de reproduction selon la revendication 5 ou d'un matériel exploitation selon la revendication 6.

11. Utilisation d'une cellule végétale génétiquement modifiée selon l'une quelconque des revendications 1 ou 2 ou d'une plante selon la revendication 3 ou 4 pour produire une farine.

12. Molécule d'acide nucléique codant pour une protéine ayant l'activité enzymatique d'une protéine qui transfère un résidu phosphate d'un nucléoside de triphosphate à l'amidon, choisie dans le groupe consistant en
a) une molécule d'acide nucléique qui code pour une protéine ayant la séquence d'acides aminés présentée dans SEQ ID N° 3 ;
b) une molécule d'acide nucléique qui code pour une protéine dont la séquence d'acides aminés a une identité d'au moins 60 % avec la séquence d'acides aminés présentée dans SEQ ID N° 3 ;
c) une molécule d'acide nucléique qui comprend la séquence nucléotidique présentée dans SEQ ID N° 1 ou SEQ ID N° 2 ou une séquence qui est complémentaire de ces séquences ;
d) molécule d'acide nucléique qui a une identité d'au moins 70 % avec les séquences d'acides nucléiques décrites en a) ou c) ;
e) une molécule d'acide nucléique qui s'hybride dans des conditions stringentes à au moins un brin de la molécule d'acide nucléique décrite en a) ou c) ;
f) une molécule d'acide nucléique dont la séquence nucléotidique, en conséquence de la dégénérescence du code génétique, s'écarte de la séquence des molécules d'acide nucléique mentionnées en a) ou c) ; et
g) une molécule d'acide nucléique qui constitue un fragment, un variant allélique et/ou un dérivé des molécules d'acide nucléique mentionnées en a), b), c), d), e) ou f),
et où ladite molécule d'acide nucléique est une molécule d'acide nucléique recombinant comprenant des séquences additionnelles qui ne sont pas naturellement présentes dans une combinaison dans laquelle elles sont présentes dans des acides nucléiques recombinants dans lesquels une séquence d'acides nucléiques codant pour une protéine qui transfère un résidu phosphate d'un nucléoside triphosphate à l'amidon est fusionnée à des séquences d'acides nucléiques qui codent pour un peptide signal plastidique.

13. Vecteur comprenant une molécule d'acide nucléique selon la revendication 12.

14. Vecteur selon la revendication 13, dans lequel la molécule d'acide nucléique est liée à des séquences régulatrices qui amorcent la transcription dans des cellules procaryotes ou eucaryotes.

15. Cellule hôte qui comprend une molécule d'acide nucléique selon la revendication 12, ou qui comprend un vecteur selon la revendication 13 ou 14.

16. Composition comprenant une molécule d'acide nucléique selon la revendication 12 ou un vecteur selon la revendication 13 ou 14.

17. Utilisation d'une composition selon la revendication 16 pour identifier des cellules végétales qui, par comparaison avec des cellules végétales de type sauvage n'ayant pas subi de modification génétique, présentent une activité accrue d'une protéine qui transfère un résidu phosphate d'un nucléoside triphosphate à l'amidon.

18. Protéine ayant une activité de phosphorylation de l'amidon, choisie dans le groupe consistant en
a) une protéine qui comprend la séquence d'acides aminés présentée dans SEQ ID N°3;
b) une protéine qui a une identité d'au moins 60 % avec la séquence d'acides aminés des protéines mentionnées en a), fusionnée à des séquences d'acides aminés qui codent pour un peptide signal plastidique.

19. Protéine recombinante qui comprend les séquences d'acides aminés codant pour une protéine selon la revendication 18, fusionnées à des séquences d'acides aminés qui codent pour un peptide signal plastidique.

20. Protéine selon la revendication 18 ou 19, qui possède un domaine phosphohistidine.
